(19) **Europäisches Patentamt European Patent Office Office européen des brevets**

(11) **EP 2 486 624 B1**

(12) **EUROPEAN PATENT SPECIFICATION**

(45) Date of publication and mention of the grant of the patent:
**25.03.2015 Bulletin 2015/13**

(51) Int Cl.:
*H01Q 1/27* $^{(2006.01)}$   *H01Q 5/00* $^{(2015.01)}$
*H01Q 9/42* $^{(2006.01)}$   *A61N 1/372* $^{(2006.01)}$

(21) Application number: **10765907.0**

(22) Date of filing: **04.10.2010**

(86) International application number:
**PCT/US2010/051369**

(87) International publication number:
**WO 2011/044062 (14.04.2011 Gazette 2011/15)**

(54) **MULTI-BAND ANTENNA FOR IMPLANTABLE DEVICE**

MEHRBANDANTENNE FÜR EINE IMPLANTIERBARE VORRICHTUNG

ANTENNE MULTIBANDE POUR DISPOSITIF IMPLANTABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.10.2009 US 248686 P**

(43) Date of publication of application:
**15.08.2012 Bulletin 2012/33**

(73) Proprietor: **Cardiac Pacemakers, Inc.**
**St. Paul, MN 55112-5798 (US)**

(72) Inventors:
• **NGHIEM, David**
**Shoreview**
**Minnesota 55126 (US)**
• **SOLFEST, Ronald, W.**
**Lino Lakes**
**Minnesota 55038 (US)**
• **MAILE, Keith, R.**
**New Brighton**
**MN 55112 (US)**
• **LARSON, Dennis, E.**
**White Bear Lake Township**
**Minnesota 55110 (US)**
• **NGUYEN, Thao**
**Eagan**
**Minnesota 55122 (US)**

(74) Representative: **Schley, Jan Malte et al**
**Fish & Richardson P.C.**
**HighLight Business Towers**
**Mies-van-der-Rohe-Str. 8**
**80807 München (DE)**

(56) References cited:
**EP-A2- 0 814 536     DE-A1- 10 049 845**
**US-A1- 2006 247 711     US-A1- 2009 182 426**
**US-A1- 2009 228 075**

EP 2 486 624 B1

**Description**

BACKGROUND

Medical devices can be implanted in a body to perform tasks including monitoring, detecting, or sensing physiological information in or otherwise associated with the body, diagnosing a physiological condition or disease, treating or providing a therapy for a physiological condition or disease, or restoring or otherwise altering the function of an organ or a tissue. Examples of an implantable medical device can include a cardiac rhythm management device, such as a pacemaker, a cardiac resynchronization therapy device, a cardioverter or defibrillator, a neurological stimulator, a neuromuscular stimulator, or a drug delivery system. In certain examples, the implantable medical device can include a telemetry circuit and an antenna, coupled to the telemetry circuit, the combination of which can be configured to provide wireless communication between the implantable medical device and an external device (e.g., to send information, such as physiological or other information, from the implantable medical device to the external device, or to receive information, such as programming instructions, at the implantable medical device from the external device).

Magnetic coupling can be used to provide short-range (e.g., centimeters) communication between an implantable medical device implanted in a body and an external device, or between an implantable medical device outside of the body and an external device. However, magnetic coupling communication largely relies on near-field radiation, where the field distribution is highly dependent upon the distance from, and orientation of, the antenna, which grossly limits the effective range of wireless communication between the implantable medical device and the external device.

As an alternative to or in addition to magnetic coupling, low power radio frequency (RF) communication, having an extended range over magnetic coupling, can be used to provide communication between an implantable medical device and an external device. However, many RF communication channels, bands, or frequencies are limited by governing bodies or other regulatory agencies, such as the Federal Communications Commission (FCC) or other regulatory body. As such, a frequency available for use in one area may not be available for use in another area.

US 2009/228075 A1 discloses a multi-band implantable telemetry system which comprises an implantable telemetry circuit coupled to an implantable antenna, wherein the implantable telemetry circuit is configured to drive the antenna in a transmit mode and receive information from the antenna in a receive mode using at least one of a first specified operating frequency or a second specified operating frequency different from the first specified operating frequency. The implantable antenna is configured to wirelessly send information electromagnetically from within a biological medium or wirelessly receive information electromagnetically in the biological medium using the first and second specified operating frequencies. The antenna includes a switchback portion comprising a first major segment having a first length, a second major segment having a second length, and a third major segment having a third length.

The first specified operating frequency is provided at a fundamental mode of the antenna, wherein the fundamental mode frequency of the antenna is different from the fundamental mode frequency of an unfolded antenna of equal total length,

US 2009/0182426 A1 refers to an implantable medical device for use in a patient management system which includes a sensor, a processor, and a first communications unit. The first communications unit can deliver notification of significant events to a host computer using a short-range telemetry first communications link. A second communications unit can deliver notification of the significant events to the host computer over a second communications link, which is over a pervasive wireless communications network, such as a cell phone network. The device includes an antenna that is operatively connected to both the first and second communications units.

Further, EP 0 814 536 A2 shows an antenna, wherein an antenna element formed by a long conductor is formed with at least one returned portion arranged substantially in parallel to a longitudinal direction of the antenna element. The physical length of the antenna element in the longitudinal direction is determined to such a length as to be substantially resonated in a first frequency band, and the at least one returned portion is formed in such a way as to be resonated in a second frequency band twice higher than the first frequency band on the basis of an electric coupling with the adjacent antenna element.

OVERVIEW

The present invention is defined by a multi-band implantable telemetry system comprising the features of claim 1 and a method comprising the features of claim 14, respectively. Preferred embodiments of the system are represented in the dependent claims.

The present inventors have recognized, among other things, a system or method for wirelessly sending information electromagnetically at one of a first or a second specified operating frequency from within a biological medium or receiving information electromagnetically at one of the first or second specified operating frequencies in the biological medium using an implantable antenna including a switchback portion having multiple segments. The first specified operating frequency and the second specified operating frequency can be provided using the multiple segments.

The following examples are not necessarily in accordance with the invention.

In Example 1, a multi-band implantable telemetry system can include an implantable telemetry circuit coupled to an implantable antenna, the implantable telemetry circuit configured to drive the implantable antenna in a transmit mode and receive information from the implantable antenna in a receive mode using at least one of a first specified operating frequency or a second specified operating frequency, the second specified operating frequency different than the first specified operating frequency, wherein the implantable antenna is configured to wirelessly send information electromagnetically from within a biological medium or wirelessly receive information electromagnetically in the biological medium using the first and second specified operating frequencies. The implantable antenna can include a switchback portion including a first major segment having a first length, a second major segment having a second length, and a third major segment having a third length, wherein the first, second, and third major segments are arranged in a switchback configuration. The first specified operating frequency is provided at a fundamental mode of the implantable antenna, wherein the fundamental mode frequency of the implantable antenna is different than the fundamental mode frequency of an unfolded antenna of equal total length, the difference provided using at least the first length of the first major segment, and the second specified operating frequency is provided at a first higher-order mode of the implantable antenna using a total length of the implantable antenna.

In Example 2, the first higher-order mode frequency of the implantable antenna of Example 1 optionally corresponds to the first higher-order mode frequency of the unfolded antenna of equal total length.

In Example 3, the first length of the first major segment of any one or more of Examples 1-2 is optionally configured to (1) optimize surface current cancellation between the first major segment and the second major segment at the fundamental mode of the implantable antenna to provide the first specified operating frequency, or (2) minimize surface current cancellation between the first major segment and the second major segment at the first higher-order mode of the implantable antenna to provide the second specified operating frequency.

In Example 4, the first length of the first major segment of any one or more of Examples 1-3 optionally corresponds to one-eighth of an effective wavelength of the first specified operating frequency.

In Example 5, each of the first length of the first major segment, the second length of the second major segment, and the third length of the third major segment of Examples 1-4 optionally separately correspond to one-eighth of the effective wavelength of the first specified operating frequency.

In Example 6, the first major segment of any one or more of Examples 1-5 is optionally located farther from a ground plane than the second major segment.

In Example 7, the first major segment of any one or more of Examples 1-6 is optionally located closer to a ground plane than the second major segment.

In Example 8, at least a portion of the first major segment of any one or more of Examples 1-7 is optionally located equidistant to a ground plane with at least a portion of the second major segment.

In Example 9, the implantable antenna of any one or more of Examples 1-8 optionally includes a wire antenna, and at least a portion of the second major segment of any one or more of Examples 1-8 is optionally separated from at least a portion of the first major segment by a first distance, wherein the first distance is larger, by at least a factor of 3, than a diameter of the wire antenna, and the first length of the first major segment is larger, by at least a factor of 5, than the first distance between the first and second major segments.

In Example 10, the switchback portion of the implantable antenna of any one or more of Examples 1-9 optionally includes a first transition between the first and second major segments, the first transition configured to couple a distal end of the first major segment to a proximal end of the second major segment, the first transition including a turn, such that the geometrical direction from a proximal end of the first major segment to the distal end of the first major segment substantially opposes the geometrical direction from the proximal end of the second major segment to a distal end of the second major segment.

In Example 11, the first and second specified operating frequencies of any one or more of Examples 1-10 are optionally selected from a list including at least one of:

    (1) a Medical Implant Communications Service (MICS) band range extending from approximately 402 MHz to approximately 405 MHz;

    (2) a Short Range Device (SRD) band range extending from approximately 862 MHz to approximately 870 MHz;

    (3) a first Industrial-Scientific-Medical (ISM) band range extending from approximately 902 MHz to approximately 928 MHz;

    (4) a second ISM band range extending from approximately 2400 MHz to approximately 2500 MHz; or

    (5) a Personal Communication Service (PCS) band range extending from approximately 1850-1990 MHz.

In Example 12, the distal end of the first major segment of any one or more of Examples 1-11 is optionally coupled to the proximal end of the second major segment, and the distal end of the second major segment of any one or more of Examples 1-11 is optionally coupled to the proximal end of the third major segment, wherein the switchback portion of the implantable antenna is composed of a single continuous conductor extending from the proximal end of the first major segment to the distal end of the third major segment.

In Example 13, the implantable antenna of any one or more of Examples 1-12 optionally includes an initial segment configured to couple the telemetry circuit to the proximal end of the first major segment, wherein the initial segment is configured to control an input impedance of the implantable antenna at least in part using a physical arrangement of at least a portion of the initial segment with respect to a return conductor to provide a substantially conjugate match in the biological medium to an output impedance of the implantable telemetry circuit.

In Example 14, the system of any one or more of Examples 1-13 optionally includes an implantable device housing including the implantable telemetry circuit and a conductive portion coupled to the implantable telemetry circuit, an implantable dielectric compartment coupled to the implantable device housing, the implantable dielectric compartment including the first, second, and third major segments of the switchback portion of the implantable antenna, the implantable dielectric compartment having a height with respect to the surface of the implantable device housing, and having a length and width along the surface of the implantable device housing, wherein the implantable antenna of any one or more of Examples 1-13 is optionally configured to wirelessly send information electromagnetically from within the implantable dielectric compartment in the biological medium or wirelessly receive information electromagnetically in the implantable dielectric compartment in the biological medium using the first and second specified operating frequencies, wherein the first, second, and third lengths of the first, second, and third major segments of any one or more of Examples 1-13 are each optionally shorter than the length of the implantable dielectric compartment, and wherein at least a portion of the second major segment of any one or more of Examples 1-13 is optionally separated from at least a portion of the first major segment by a first distance, the first distance shorter than at least one of the height or width of the implantable dielectric compartment.

In Example 15, at least one of the first, second, or third major segments of the switchback portion of the implantable antenna of any one or more of Examples 1-14 are optionally located proximate at least one of a side or a top of the implantable dielectric compartment.

In Example 16, a multi-band implantable telemetry system includes an implantable telemetry circuit coupled to an implantable antenna, the implantable telemetry circuit configured to drive the implantable antenna in a transmit mode and receive information from the antenna in a receive mode using one of a first specified operating frequency in a Medical Implant Communications Service (MICS) band frequency range extending from approximately 402 MHz to approximately 405 MHz, or a second specified operating frequency in an Industrial-Scientific-Medical (ISM) band frequency range extending from approximately 902 MHz to approximately 928 MHz. The implantable antenna is configured to wirelessly send information electromagnetically from within an implantable dielectric compartment in a biological medium and to wirelessly receive information electromagnetically in the implantable dielectric compartment in the biological medium using the first and second specified operating frequencies, the implantable antenna including a switchback portion, the switchback portion including a first major segment having a first length, a second major segment having a second length, and a third major segment having a third length, wherein the first, second, and third major segments are arranged in a switchback configuration. In Example 15, the first specified operating frequency is provided at a fundamental mode of the implantable antenna, wherein the fundamental mode frequency of the implantable antenna is different than a fundamental mode frequency of an unfolded antenna of equal total length, the difference provided using at least the first length of the first major segment and the second specified operating frequency is provided at a first higher-order mode of the implantable antenna using a total length of the implantable antenna, wherein, using the first length of the first major segment, the switchback portion of the implantable antenna is configured to (1) optimize surface current cancellation between the first major segment and the second major segment at the fundamental mode of the implantable antenna to provide the first specified operating frequency, or (2) minimize surface current cancellation between the first major segment and the second major segment at the first higher-order mode of the implantable antenna to provide the second specified operating frequency, wherein each of the first length of the first major segment, the second length of the second major segment, and the third length of the third major segment separately correspond to one-eighth of the effective wavelength of the first specified operating frequency.

In Example 17, the first higher-order mode frequency of the implantable antenna of Example 16 optionally corresponds to the first higher-order mode frequency of the unfolded antenna of equal total length.

In Example 18, the system of any one or more of Examples 16-17 optionally includes an initial segment configured to couple the telemetry circuit to a proximal end of the first major segment, and wherein the initial segment is configured to control an input impedance of the implantable antenna at least in part using a physical arrangement of at least a portion of the initial segment with respect to a return conductor to provide a substantially conjugate match in the biological medium to an output impedance of the implantable telemetry circuit.

In Example 19, a method includes driving an implantable antenna in a transmit mode and receiving information from the implantable antenna in a receive mode using at least one of a first specified operating frequency or a second specified operating frequency different than the first specified operating frequency, wirelessly sending information electromagnetically at one of the first or second specified operating frequencies using the implantable antenna from within a biological medium or wirelessly receiving information electromagnetically at one of the first or the second specified operating frequencies using the implantable antenna in the biological medium, the implantable antenna including a switchback portion, the switchback portion including a first major segment having a first length, a second major segment having a second length, and a third major segment having a third length, the first, second, and third major segments arranged in a switchback configuration, providing the first specified operating frequency at a fundamental mode of the implantable antenna, wherein the fundamental mode frequency of the implantable antenna is different than the fundamental mode frequency of an unfolded antenna of equal total length, the difference provided at least using the first length of the first major segment, and providing the second specified operating frequency at a first higher-order mode of the implantable antenna using a total length of the implantable antenna.

In Example 20, the driving or the receiving information from the implantable antenna of Example 19 includes optionally using the first specified operating frequency in one of or between a Medical Implant Communications Service (MICS) band frequency range extending from approximately 402 MHz to approximately 405 MHz, or using the second specified operating frequency in an Industrial-Scientific-Medical (ISM) band frequency range extending from approximately 902 MHz to approximately 928 MHz and a Short Range Device (SRD) band range extending from approximately 862 MHz to approximately 870 MHz.

In Example 21, the providing the second specified operating frequency at the first higher-order mode of the implantable antenna of any one or more of Examples 19-20 optionally includes providing the second specified operating frequency at the first higher-order mode corresponding to the first higher-order mode frequency of the unfolded antenna of equal total length.

In Example 22, the method of any one or more of Examples 19-21 optionally include optimizing surface current cancellation between the first major segment and the second major segment at the fundamental mode of the implantable antenna using the first length of the first major segment to provide the first specified operating frequency, and minimizing surface current cancellation between the first major segment and the second major segment at the first higher-order mode of the implantable antenna using the first length of the first major segment to provide the second specified operating frequency.

In Example 23, the using the first length of the first major segment of any one or more of Examples 19-22 optionally includes using a length corresponding to one-eighth of an effective wavelength of the first specified operating frequency.

In Example 24, the method of any one or more of Examples 19-23 optionally includes coupling a telemetry circuit to the switchback portion of the implantable antenna using an initial segment, and controlling an input impedance of the implantable antenna at least in part using a physical arrangement of at least a portion of the initial segment with respect to a return conductor to provide a substantially conjugate match in the biological medium to an output impedance of the implantable telemetry circuit.

This overview is intended to provide an overview of subject matter of the present patent application. It is not intended to provide an exclusive or exhaustive explanation of the invention. The detailed description is included to provide further information about the present patent application.

BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various examples discussed in the present document.

FIG. 1 illustrates generally an example of a system including an implantable antenna coupled to an implantable telemetry circuit.

FIG. 2 illustrates generally an example of a system including an implantable device wirelessly coupled to an external module.

FIGS. 3A-3C illustrate generally examples of systems including an implantable antenna, an initial segment, a feedthrough, and a ground plane.

FIG. 4A illustrates generally an example of an unfolded dipole antenna.

FIGS. 4B-4C illustrate generally examples of surface current distributions along an unfolded dipole antenna.

FIG. 5A illustrates general an example of a three major segment folded dipole antenna.

FIGS. 5B-5C illustrate generally examples of surface current distributions along a three major segment folded dipole antenna.

FIG. 6 illustrates generally an example of a relationship between a return loss (in decibels) and frequency (in GHz) of two example antenna configurations.

FIG. 7 illustrates generally an example of a system including an implantable antenna having a switchback portion including a first major segment, a second major segment, and a third major segment.

FIG. 8 illustrates generally an example of a relationship between a return loss (in decibels) and frequency (in GHz) of the antenna configuration illustrated in FIG. 7.

FIG. 9 illustrates generally an example of a system including an implantable antenna having a switchback portion.

FIG. 10 illustrates generally an example of a relationship between a return loss (in decibels) and frequency (in GHz) of the antenna configuration illustrated in FIG. 9.

FIG. 11 illustrates generally an example of a system including an implantable antenna having a switchback portion.

FIG. 12 illustrated generally an example of a relationship between a return loss (in decibels) and frequency (in GHz) of the antenna configuration illustrated in FIG. 11.

FIG. 13A illustrates generally an example of a two major segment folded dipole antenna.

FIGS. 13B-13C illustrate generally examples of surface current distributions along a two major segment folded dipole antenna.

FIG. 14 illustrates generally an example of a relationship between a return loss (in decibels) and frequency (in GHz) of two example antenna configurations.

FIG. 15 illustrates generally an example of a method including wirelessly transferring information electromagnetically at different first or second specified operating frequencies.

## DETAILED DESCRIPTION

An antenna can be configured to transmit or receive electromagnetic waves, for example, by converting electrical current into electromagnetic waves, or by converting electromagnetic waves into electrical current. A dipole antenna typically includes two conductors having approximately equal lengths extending from a common or similar feed point. The length of one-half of an unfolded dipole antenna typically corresponds to one-quarter of the wavelength of the fundamental mode frequency of the unfolded dipole antenna.

For example, in air (or a medium having an effective dielectric constant of approximately 1), the length of a dipole antenna having a desired fundamental mode frequency of 1 GHz generally corresponds to one-half of the 1 GHz wavelength, or approximately 15 cm. For an unfolded half-wavelength dipole antenna, resonance typically repeats at odd multiples of the fundamental mode frequency. Thus, for the 15cm long dipole antenna having a fundamental mode frequency at 1 GHz, a first higher-order mode frequency can appear at 3 GHz, a second higher-order mode frequency can appear at 5 GHz, etc.

Generally, the desired length of an antenna changes roughly inversely proportionately to the square root of the effective dielectric constant of the medium surrounding the antenna. Thus, for a desired fundamental mode frequency, the desired antenna length decreases as the effective dielectric constant of the transmission medium increases. In an example, the antenna can be configured to be implanted in a biological medium (e.g., tissue, blood, muscle, one or more materials simulating a biological medium, etc.). In certain examples, the antenna in the biological medium can be surrounded by a polymer or other dielectric material of varying thickness, affecting the effective dielectric constant seen by the antenna.

Another type of antenna is a monopole antenna. Generally, a monopole antenna includes one-half of the dipole antenna proximate a ground plane. If the ground plane is large enough, the monopole antenna can behave similar to the dipole antenna, using reflection from the ground plane to account or make up for the second half of the dipole antenna. Accordingly, the length of an unfolded monopole antenna typically corresponds to one-quarter of the wavelength of the fundamental mode frequency of the unfolded monopole antenna.

In certain examples, it can be desirable to communicate at more than one specified operating frequency, for example, to provide operating capability in multiple geographic regions, to provide operating compatibility with various systems or devices, to provide backup wireless capability (e.g., due to interference, poor propagation characteristics, malfunction, high data error rate, etc.), or for one or more other reasons. Generally, basic antennas (e.g., the unfolded monopole antenna, the unfolded dipole antenna, etc.) can be configured to communicate at the fundamental mode of the antenna, as well as at higher-order modes of the antenna. However, resonance of these antennas is generally limited to the fundamental mode frequency and to odd multiples (higher-order mode frequencies) of the fundamental mode frequency.

The present inventors have recognized, among other things, that it can be desirable to communicate using a single antenna at more than one specified operating frequency different than the fundamental mode frequency or one or more higher-order mode frequencies of the antenna. For example, it can be advantageous to communicate at a first operating frequency in a Medical Implant Communications Service (MICS) band range (e.g., 402-405 MHz) and at a second operating frequency in an Industrial-Scientific-Medical (ISM) band range (e.g., 902-928 MHz), at a first operating frequency in a MICS band range and at a second operating frequency in a Global Positioning System (GPS) band range (e.g., L1, 1572-1578 MHz), or at first and second operating frequencies in a combination of one or more other frequency band ranges (e.g., a second ISM band range (e.g., 2400-2500 MHz), an L2 GPS band range (e.g., 1224-1230 MHz), a Personal Communication Service (PCS) band range (e.g., 1850-1990 MHz), etc.).

Generally, the surface current distribution along an antenna varies with frequency. The present inventors have recognized, among other things, that bending, folding, or otherwise altering the antenna along its length (e.g., using one or more transitions, such as a switchback portion) can change the current distribution along the antenna (e.g., with respect to the current distribution along the unfolded antenna of similar total length). Moreover, the present inventors have recognized that, by changing the current distribution along the length of the antenna (e.g., using the one or more transitions), at least one of the fundamental mode frequency or one or more higher-order mode frequencies can be adjusted to a desired operating frequency different than the original fundamental mode frequency or the one or more higher-order mode frequencies of the implantable antenna.

In an example, the fundamental mode can be altered while one or more of the higher-order modes remain substantially unchanged (e.g., with respect to the higher-order modes of an unfolded antenna of similar total length). In other examples, both of the fundamental mode and one or more of the higher-order modes can be altered.

In an example, to adjust an antenna to resonate at a first operating frequency and at a second operating frequency (the second operating frequency larger than the first operating frequency), the present inventors have recognized that a total length of the antenna can be selected such that an unfolded antenna of similar total length would resonate at or near the second desired operating frequency using a higher-order mode of the unfolded antenna (e.g., the fundamental mode frequency established as a function of the total length of the unfolded antenna). Further, in this example, the present inventors have recognized that the fundamental mode frequency of the unfolded antenna can be altered (e.g., using at least a first transition along the length of the antenna, changing the current distribution of the antenna with respect to the unfolded antenna of similar total length) to resonate at or near the first desired operating frequency (e.g., the first desired operating frequency different than the fundamental mode frequency of the unfolded antenna), while substantially resonating at or near the second desired frequency using the higher-order mode.

Generally, for a given antenna length, the fundamental mode frequency and the higher-order mode frequencies of a similar length unfolded antenna can be estimated, simulated, or otherwise established. Thus, for a set of desired operating frequencies, a total length of the antenna can be selected to provide a desired operating frequency corresponding to (being similar or equivalent to) a higher-order mode frequency of the antenna (e.g., near one of the higher-order mode frequencies of the antenna, such that an adjustment to the fundamental mode frequency or the higher-order mode frequency to the desired operating frequency can be made). In certain examples, one or more transitions (e.g., one or more major segments in a switchback portion) can be placed along the length of the antenna altering surface current cancellation patterns for the fundamental mode or for one or more higher-order modes to optimize the resulting resonant frequencies with respect to the desired operating frequencies.

In an example, depending on the desired operating frequencies, the one or more transitions can be placed to minimize the effect of the transitions for the second desired operating frequency (the second desired operating frequency larger than the first desired operating frequency) (e.g., minimize the change in the current distribution, minimize the surface current cancellation between major segments of a switchback portion, etc., to minimize the affect on the one or more higher-order mode frequencies as compared to an unfolded antenna of similar total length). In other examples, the one or more transitions can be placed to optimize the effect of the transitions for the first desired operating frequency (e.g., optimize the change in the current distribution, optimize the surface current cancellation between major segments of the switchback portion, etc., to optimize the affect on the fundamental mode frequency or of one or more higher-order mode frequencies with respect to an unfolded antenna having a similar total length).

In an example, a first desired operating frequency and a second desired operating frequency in an implantable monopole antenna can be provided by selecting a total length for the implantable antenna roughly corresponding to one-quarter wavelength of a fundamental mode frequency having the second desired operating frequency as a higher-order mode frequency. In this example, one or more transitions can be placed along the length of the implantable antenna (e.g., changing the current distribution along the implantable antenna) to optimize adjustment of the fundamental mode frequency towards the first desired operating frequency and minimize adjustment of the higher-order mode to retain the higher-order mode frequency near the second desired operating frequency. In other examples, both of the fundamental mode and one or more of the higher-order modes of the implantable antenna can be optimized to move the fundamental mode frequency and the one or more higher-order mode frequencies towards the first and second desired operating frequencies using the one or more transitions.

In an example, using the one or more transitions, the direction of the surface current between multiple segments of a switchback portion of an implantable antenna can be used to optimize a fundamental mode, one or more higher-order mode, or both of the fundamental mode and the one or more higher-order mode with respect to one or more desired operating frequencies. In other examples, using the one or more transitions, the direction of the surface current along a single segment of the switchback portion of the implantable antenna can be used to optimize the fundamental mode, the one or more higher-order mode, or both of the fundamental mode and the one or more higher-order mode with respect to the one or more desired operating frequencies. In other examples, one or more other characteristics of the antenna configuration can be used to optimize the effect of the one or more transitions, such as the spacing between various portions of subsequent or other segments of the switchback portions, the distance between one or more return conductors (e.g., a ground plane, a conductive housing, etc.) in the implantable dielectric compartment, the total length of the implantable antenna, the length of one or more of the segments of the switchback portion, etc.

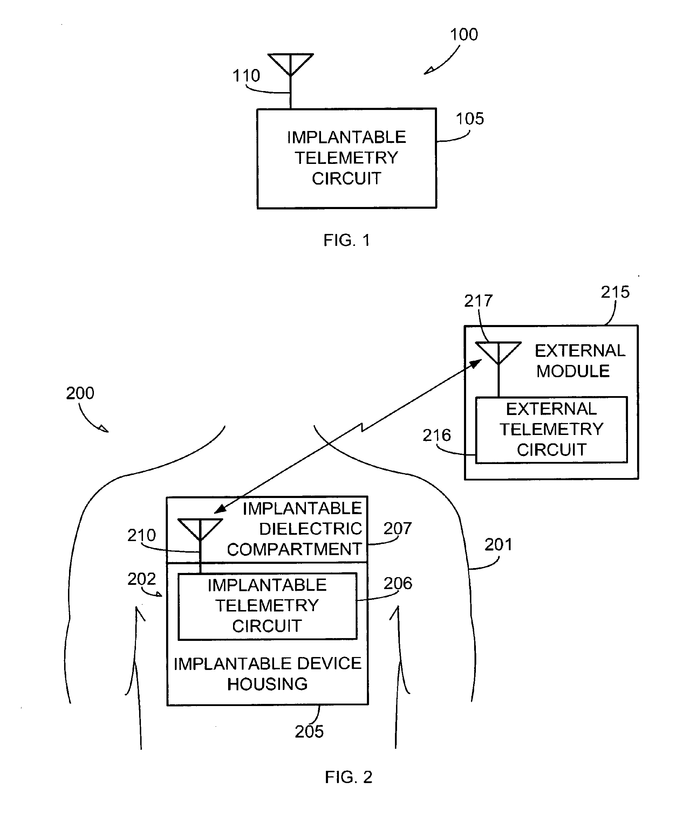

FIG. 1 illustrates generally an example of a system 100 including an implantable antenna 110 coupled to an implantable telemetry circuit 105.

In an example, the implantable telemetry circuit 105 can be configured to drive the implantable antenna 110 in a transmit mode and to receive information from the implantable antenna 110 in a receive mode using one of a first specified operating or a second specified operating frequency. In an example, the first and second specified operating frequencies can be separated by at least an octave, but not directly harmonically related (e.g., a higher desired operating frequency is not an odd multiple of a lower desired operating frequency). In certain examples, the system 100 can include multiple implantable telemetry circuits configured to drive or receive information from the implantable antenna 110.

In an example, the implantable antenna 110 can be configured to wirelessly send information electromagnetically from within a biological medium or wirelessly receive information electromagnetically in the biological medium using at least one of the first or second specified operating frequencies. In an example, the implantable antenna 110 can be configured to wirelessly transfer (e.g., send or receive) information electromagnetically using multiple (e.g., two or more) frequencies at the same time.

In an example, the implantable antenna 110 can include one or more different antenna configurations formed from a single conductor including a switchback portion. In an example, the switchback portion of the implantable antenna 110 can have a total length corresponding to approximately one-quarter of a wavelength of one of the first or second specified operating frequency in the biological medium. In an example, the implantable antenna 110 can include a dipole antenna, a monopole antenna, an inverted-F antenna, an inverted-L antenna, or one or more other antenna configurations.

FIG. 2 illustrates generally an example of a system 200 including an implantable device 202 wirelessly coupled to an external module 215. The implantable device 202 includes an implantable device housing 205 including an implantable telemetry circuit 206, and an implantable dielectric compartment 207 (e.g., a header) coupled to the implantable device housing 205, the implantable dielectric compartment 207 including at least a portion of an implantable antenna 210. In an example, the implantable antenna 210 can be coupled to the implantable telemetry circuit 206 using an initial segment. The initial segment can be included as a portion of the implantable antenna 210, adding to the total length of the implantable antenna 210. Further, at least a portion of the implantable device housing 205 can include a conductor configured to provide a ground plane for the implantable antenna 210.

In an example, the implantable device 202 can include a pacemaker, a defibrillator, or one or more other implantable medical devices.

In an example, the external module 215 can include an external telemetry circuit 216 and an external antenna 217 (e.g., or one or more external antennae). In an example, the external module 215 can include a local medical device programmer or other local external module (e.g., a medical device programmer or other external module within wireless communication of the implantable antenna 210). In other examples, the external module 215 can include a remote medical device programmer or one or more other remote external modules (e.g., outside of wireless communication range of the implantable antenna 210, but coupled to the implantable device 202 using a local external device, such as a repeater).

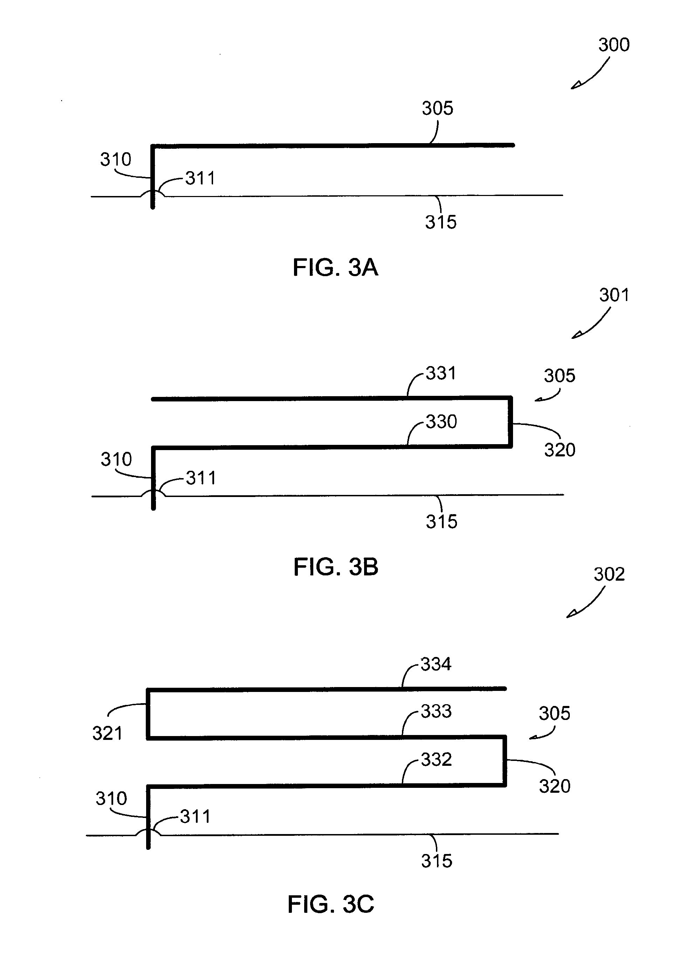

FIGS. 3A-3C illustrate generally examples of systems 300-302 including an implantable antenna 305, an initial segment 310, a feed-through 311, and a ground plane 315. In an example, the implantable antenna can include at least one of a conductive wire or a conductive strip. In an example, the initial segment 310 can be configured to couple the implantable antenna 305 to an implantable telemetry circuit (e.g., the implantable telemetry circuit 206). In an example, the implantable telemetry circuit can be located in an implantable device housing (e.g., the implantable device housing 205), and the initial segment 310 can be configured to couple the implantable antenna 305, at least a portion located outside of the implantable device housing (but within an implantable dielectric compartment), to the implantable telemetry circuit through the feed-through 311. In an example, the feed-through 311 can prevent the implantable device housing from attenuating, shorting out, or otherwise altering the radiation of electromagnetic energy from the implantable antenna 305.

FIG. 3A illustrates generally an example of a system 300 including an implantable antenna 305 and an initial segment 310, the implantable antenna 305 located over a ground plane 315. In an example, the ground plane 315 can include one or more conductors configured to reflect at least a portion of the electromagnetic radiation to or from the implantable antenna 305. In certain examples, the ground plane can include at least a portion of an implantable device housing (e.g., implantable device housing 205), or one or more other conductors or ground planes.

In FIG. 3A, the implantable antenna 305 includes a monopole antenna positioned over a ground plane 315 in an inverted-L configuration. In this example, the implantable antenna 305 does not include a switchback portion. Accordingly, the implantable antenna 305 can be configured to transfer information electromagnetically at a fundamental mode frequency established as a function of the length of the implantable antenna 305 (including an initial segment) in the implantable dielectric compartment in the biological medium. Further, the implantable antenna 305 can be configured to transfer information electromagnetically at one or more higher-order modes (e.g., at odd multiples of the fundamental mode frequency).

FIG. 3B illustrates generally an example of a system 301 including an implantable antenna 305 and an initial segment 310, the implantable antenna 305 located over a ground plane 315. In contrast to the example of FIG. 3A, the implantable antenna 305 of FIG. 3B includes a switchback portion having a first major segment 330 and a second major segment 331.

In an example, the implantable antenna 305 can include a first transition 320 configured to couple the first major segment 330 to the second major segment 331. In certain examples, the first transition 320 can include a bend, a curve, a straight segment, or one or more other transitions from the first major segment 330 to the second major segment 331. In an example, the first transition can be configured to alter the surface current distribution along the implantable antenna 305.

In an example, the length of the first major segment can correspond to a portion of an effective wavelength of a fundamental mode frequency of the implantable antenna 305 (e.g., to one-eighth of the effective wavelength of the fundamental mode frequency, or one or more other lengths, depending on the desired operating frequencies). In the example of FIG. 3B, the length of the first transition 320 is much shorter than the length of the first major segment 330 (e.g., by at least a factor of 5). In certain examples, the length of the first transition 320 can have a minimum length of approximately three times the diameter or width of the implantable antenna 305 (e.g., a wire diameter, a strip width, etc.). In certain examples, the length of the first major segment 330 or the first transition 320 can be limited by the size (e.g., one or more of a height, length, or width) of an implantable dielectric compartment (e.g., the implantable dielectric compartment 207) housing the implantable antenna 305.

In an example, if the length of each major segment is short (e.g., substantially equal to the length of the first transition 320), surface current cancelation between the first major segment 330 and the second major segment 331 can occur, reducing the radiation efficiency of the implantable antenna 305. However, because the lengths of the first major segment 330 and the second major segment 331 are long in terms of wavelength, the radiation efficiency of the implantable antenna 305 is less affected due to the difference in magnitude of the surface current at the overlapping segment portions. For example, in the example of FIG. 3B, at the proximal end of the first major segment 330 (near the initial segment 310), the magnitude of the surface current is high. However, because at the overlapping portion of the second major segment 331, the distal end of the second major segment 331, the magnitude of the surface current is lower (e.g., zero or near zero), the amount of surface current cancelation between the two points is minimal. If the length of each major segment was shorter, the amount of surface current cancelation in the implantable antenna would be greater, due to the likely higher magnitude of surface current at the overlapping portions.

Further, the physical orientation of the multiple major segments can affect the surface current cancelation between them. In an example, at least a portion of the first major segment 330 can be parallel to at least a portion of the second major segment 331 (or to one or more other segments). However, parallel segments can be more affected by surface current cancellation. In other examples, at least a portion of the first major segment 330 can be anti-parallel to at least a portion of the second major segment 331. In an example, anti-parallel segments can be less affected by surface current cancellation. Further, the spacing between major segments can affect the surface current cancellation in the switchback portion. In certain examples, the smaller the distance between the major segments, the greater the potential for surface current cancellation between them, due to the proximity of the cancellation currents.

In certain examples, one or more of the major segments of the switchback portion of the implantable antenna 305 can include a straight or substantially straight segment. In other examples, the first major segment 330 can be the same length, or substantially the same length, as the second major segment 331. In an example, the first major segment 330 and the second major segment 331 can be proportional to the same effective operating frequency wavelength in the biological medium, but slightly different in actual length. In other examples, one or more of the lengths of the first major segment 330 or the second major segment 331 can be increased or decreased to efficiently communicate at the first or second specified operating frequencies.

FIG. 3C illustrates generally an example of a system 302 including an implantable antenna 305 and an initial segment 310, the implantable antenna 305 located over a ground plane 315. The implantable antenna 305 of FIG. 3C includes a switchback portion having a first major segment 332, a second major segment 333, and a third major segment 334. In an example, the implantable antenna 305 can include a first transition 320 configured to couple the first major segment 332 to the second major segment 333, and a second transition 321 configured to couple the second major segment 333 to the third major segment 334. In an example, at the fundamental mode frequency or one or more higher-order mode frequencies, the second transition 321 can be configured to alter the surface current distribution along the implantable antenna 305, however, less than the first transition 320, due at least in part to the magnitude of the surface current at the second transition 321 being lower than at the first transition 320.

In an example, the lengths of the second major segment 333 and the third major segment 334 can be configured to provide the remainder of the total length of the switchback portion of the implantable antenna 305 following the first major segment 332. In certain examples, due to the surface current cancellation between multiple segments of the switchback portion of the implantable antenna 305, the total length of the switchback portion or the total length of the implantable antenna 305 can extend beyond that required for a quarter-wavelength unfolded antenna. In certain examples, the number of segments in the switchback portion can be dependent on the length available for the implantable antenna (e.g., when the implantable antenna resides in an implantable dielectric compartment, the number of segments in the switchback portion can depend on a total or useable length of the implantable dielectric compartment). In various examples, the number of segments in the switchback portion can include more, or less, than the three segments illustrated in the example of FIG. 3C. Accordingly, the length of the segments can depend on the number of segments in the switchback portion, as well as the one or more desired operating frequencies.

In an example, the implantable antenna 305 can include a switchback (e.g., zigzag, folded, etc.) portion having three major segments. In certain examples, each of the three major segments can correspond to one-eighth of an effective wavelength of a lower desired operating frequency, and the total length of the implantable antenna 305 can correspond to one-quarter of an effective wavelength of a fundamental mode frequency having a higher-order mode frequency at or near a higher desired operating frequency. The length of the first major segment 332 can be configured to alter the fundamental mode frequency of the implantable antenna while substantially retaining one or more of the higher-order mode frequencies.

In an example, the three major segments can include substantially linear antenna segments having slightly or substantially different lengths, depending on the desired operating frequencies. Further, one or more of the three major segments can be parallel to one or more other major segment, or can be the same or a different length as one or more other major segment, depending on the desired operating frequencies.

In other examples, the geometrical direction of the first major segment 332 of the switchback portion of the implantable antenna 305 can substantially oppose the geometrical direction of the second major segment 333 of the implantable antenna. In an example, a geometrical direction from the proximal end of the first major segment 332 to the distal end of the first major segment 332 can substantially oppose a geometrical direction from the proximal end of the second major segment 333 to the distal end of the second major segment 333 (e.g., oppose by 180 degrees, 135 degrees, etc.).

Generally, factors that can affect the ability of the implantable antenna 305 to achieve a first or second desired operating frequency ranges include the total length of the implantable antenna 305, the total number of segments in the switchback portion of the implantable antenna 305, the length of the switchback segments, the spacing between switchback segments, etc.

In an example, an implantable antenna can be configured to wirelessly transfer information electromagnetically using a first specified operating frequency in a MICS band frequency range (e.g., 402 - 405 MHz) and a second specified operating frequency in an ISM band frequency range (e.g., 902-928 MHz).

In an example, the ISM band frequency can be provided using a higher-order mode of the implantable antenna. In an example, the fundamental mode frequency of the implantable antenna can correspond to a total length of the implantable antenna (e.g., one-quarter of an effective wavelength of the fundamental mode frequency of the implantable antenna). In an example, the total length can be chosen such that a first higher-order mode frequency (e.g., approximately three times the fundamental mode frequency) can roughly correspond to the ISM band frequency.

Further, the present inventors have recognized, among other things, that one or more transitions placed along the length of the implantable antenna, defining one or more switchback portions, can be configured to alter, adjust, or detune the fundamental mode frequency of the implantable antenna to provide a desired operating frequency at the fundamental mode different than a fundamental mode frequency of an unfolded antenna of similar total length.

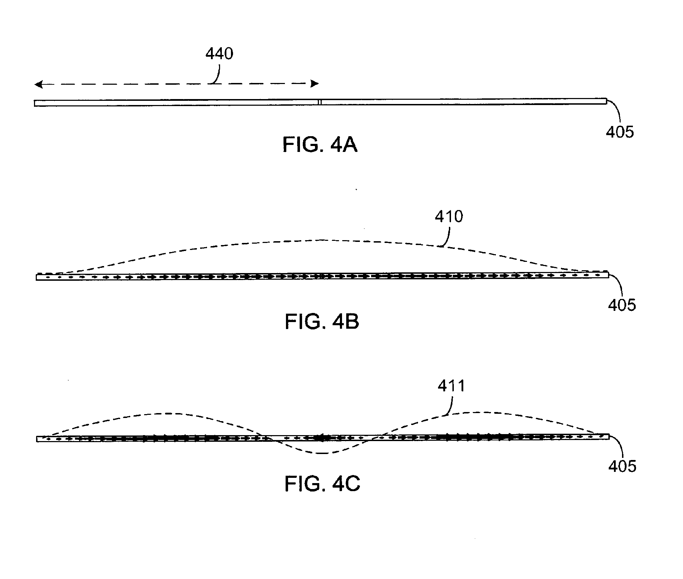

FIG. 4A illustrates generally an example of an unfolded dipole antenna 405 including two segments, each segment having a first length 440 corresponding to one-quarter of an effective wavelength of a fundamental mode frequency.

In an example, the fundamental mode frequency of the unfolded dipole antenna 405 can provide a higher-order mode frequency near a desired operating frequency (e.g., a first higher-order mode frequency, or other higher-order mode frequency, depending on the number of segments or the desired operating frequencies). In an example, an unfolded antenna having a fundamental mode frequency of approximately 305 MHz can include a first higher-order mode frequency near 915 MHz, the center frequency of the ISM band frequency range.

FIG. 4B illustrates generally an example of a first surface current distribution 410 along an unfolded dipole antenna 405 at a fundamental mode of the unfolded dipole antenna 405. The first surface current distribution 410 at the fundamental mode alternates, having a maximum at the feed point of the unfolded dipole antenna 405 and falling towards zero at or near the end of each antenna segment.

FIG. 4C illustrates generally an example of a second surface current distribution 411 along an unfolded dipole antenna 405 at a first higher-order mode of the unfolded dipole antenna 405. The second surface current distribution 411 at the higher-order mode alternates, though different than the first surface current distribution 410 at the fundamental mode of the unfolded dipole antenna 405.

In certain examples, because the second surface current distribution 411 is different than the first surface current distribution 410, a transition in the unfolded dipole antenna 405 can affect the first surface current distribution 410 differently than second surface current distribution 411. In an example, one or more transitions can be configured to alter a surface current distribution of the fundamental mode of the unfolded dipole antenna 405 and differently alter or leave substantially unaltered the surface current distribution at one or more higher-order modes of the unfolded dipole antenna 405.

In an example, the one or more transitions can be configured to optimize the surface current distribution at a fundamental mode, changing the fundamental mode frequency to provide a first specified operating frequency different than a fundamental mode frequency of an unfolded antenna of a similar total length, while minimizing changes to a higher-order mode to retain a second specified operating frequency with respect to a higher-order mode frequency of the unfolded antenna of similar total length. In other examples, the one or more transitions can be configured to optimize the surface current distribution at both a fundamental and at one or more higher-order modes to provide the first and second desired operating frequencies.

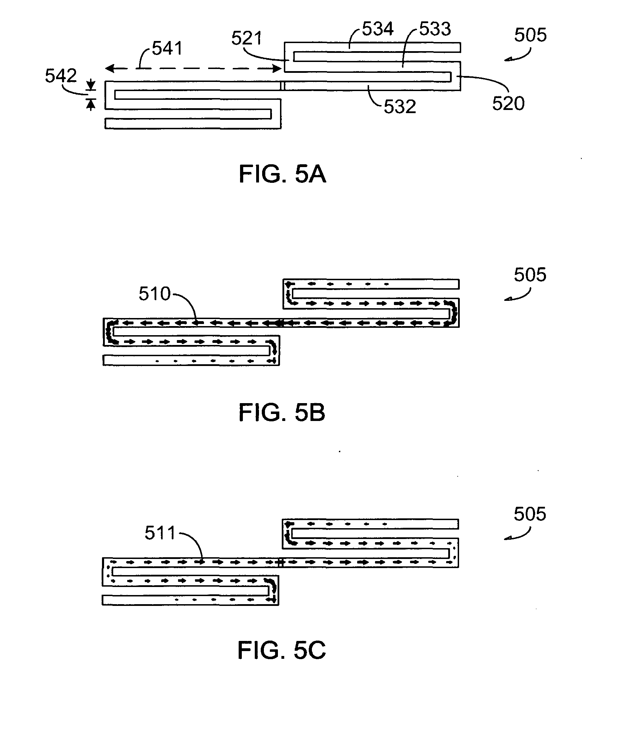

FIG. 5A illustrates generally an example of a three-segment folded dipole antenna 505 (such as illustrated in the example of FIG. 4A) including one or more transitions defining a switchback portion. In the example of FIG. 5A, the switchback portion includes a first major segment 532, a second major segment 533, and a third major segment 534, the first major segment 532 coupled to the second major segment 533 using a first transition 520, and the second major segment 533 coupled to the third major segment 534 using a second transition 521. In an example, each major segment can have a second length 541, and each transition can have a third length 542. In an example, the first length 440 of FIG. 4A can relate to the second length 541 and the third length 542 of FIG. 5A, where:

$$\text{the first length } 440 \cong (3 \text{ x the second length } 541) + (2 \text{ x the third length } 542).$$

In other examples, one or more of the major segments or transitions can include one or more other lengths, depending on the desired operating frequencies.

In an example, a dipole antenna as illustrated in FIG. 4A can have a fundamental mode frequency including higher-order mode frequency (e.g., a first higher-order mode frequency) in the ISM band frequency range. As illustrated in FIG. 5A, one or more transitions can be placed along the length of the three-segment folded dipole antenna 505 to alter the fundamental mode of the three-segment folded dipole antenna 505. In an example, to provide a desired operating frequency in the MICS band frequency range, the first transition 520 can be placed such that the length of the first major segment 532 corresponds to one-eighth of the effective wavelength of the desired operating frequency. In an example, each of the first, second, and third major segments can separately correspond to one-eighth of the effective wavelength of the desired operating frequency range.

FIG. 5B illustrates generally an example of a first surface current distribution 510 along a three-segment folded dipole antenna 505 at a fundamental mode of the three-segment folded dipole antenna 505.

FIG. 5C illustrates generally an example of a second surface current distribution 511 along a three-segment folded dipole antenna 505 at a first higher-order mode of the three-segment folded dipole antenna 505.

As illustrated in the example of FIG. 5B, at a fundamental mode, the direction of the first surface current distribution 510 in a first major segment of the three-segment folded dipole antenna 505 generally opposes the direction of the first surface current distribution 510 in a second major segment of the three-segment folded dipole antenna 505. Accordingly, surface current cancellation can occur, affecting the fundamental mode of the three-segment folded dipole antenna 505.

However, as illustrated in the example of FIG. 5C, at the first higher-order mode, the direction of the second surface current distribution 511 in a first major segment of the three-segment folded dipole antenna 505 does not substantially oppose the direction of the second surface current distribution 511 in a second major segment of the three-segment folded dipole antenna 505. Here, less surface current cancellation can occur, affecting the first higher-order mode of the three-segment folded dipole antenna 505 less than the fundamental mode.

Accordingly, the switchback configuration of FIG. 5A affects the fundamental mode more than the first higher-order mode.

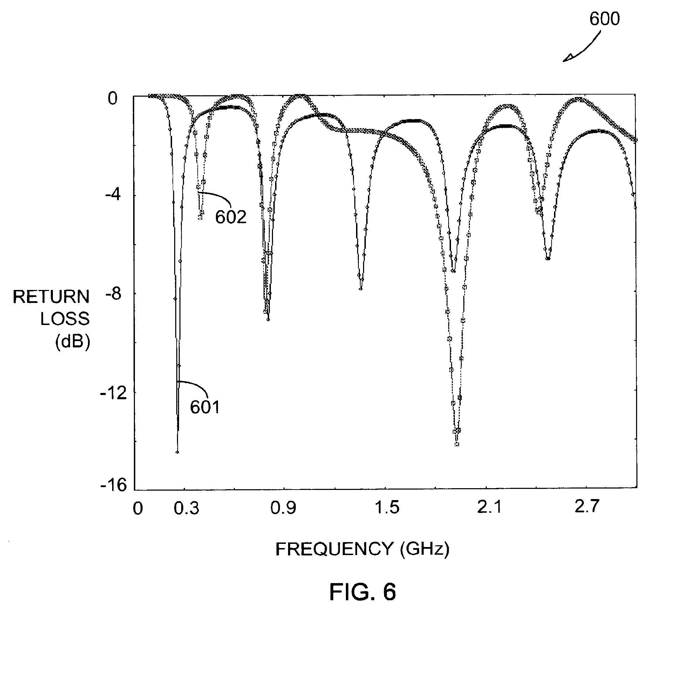

FIG. 6 illustrates generally an example of a relationship 600 between a return loss (in decibels) and frequency (in GHz) of two example antenna configurations. In this example, plot 601 illustrates generally an example of return loss characteristics of an unfolded dipole antenna (e.g., such as shown in FIG. 4A) having a fundamental mode frequency at approximately 300 MHz. Plot 601 illustrates generally that higher-order mode frequencies, in this example, appear at odd multiples of the fundamental mode frequency (e.g., at approximately 900 MHz, at approximately 1.5 GHz, 2.1 GHz, 2.7 GHz, etc.). Further, in this example, plot 602 illustrates generally an example of a folded dipole antenna having a switchback portion (e.g., such as shown in FIG. 5A) having a higher-order mode frequencies at approximately 900 MHz, but having a fundamental mode frequency at approximately 400 MHz.

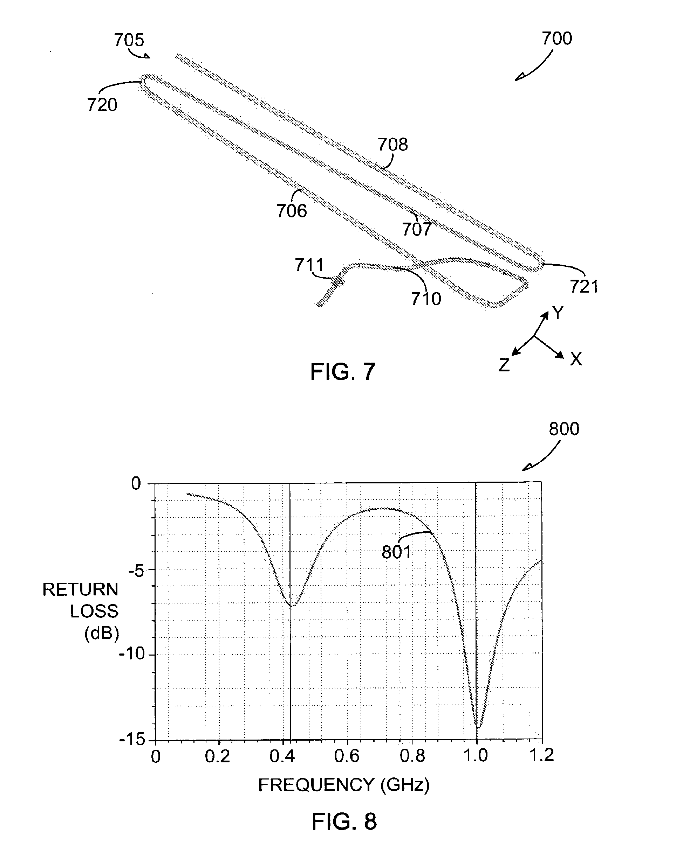

FIG. 7 illustrates generally an example of a system 700 including an implantable antenna 705 having a switchback portion including a first major segment 706, a second major segment 707, and a third major segment 708. In certain examples, the implantable antenna can include a first transition 720 configured to couple the first major segment 706 to the second major segment 707, and a second transition 721 configured to couple the second major segment 707 to the third major segment 708. In an example, the implantable antenna 705 can include an initial segment 710 different from the first major segment 706 of the switchback portion of the implantable antenna 705, the initial segment 710 configured to couple the first major segment 706 to an implantable telemetry circuit in an implantable device housing through feed-through 711.

In certain examples, the feed-through 711 can be located a distance from the first major segment 706. In certain examples, the implantable antenna 705 can include a single continuous conductor, such as a single piece of wire (e.g., having a diameter of 0.381mm), extending from the proximal end of the first major segment 706 to the distal end of the third major segment 708, or from the proximal end of the initial segment 710 to the distal end of the third major segment 708.

In certain examples, when included, the length or shape of the initial segment 710 can be selected to tune the implantable antenna 705 to a desired operating frequency, to affect an input impedance of the implantable antenna 705, or to otherwise change, alter, or affect one or more antenna characteristics. In an example, a physical arrangement can be provided between at least a portion of the initial segment 710 with respect to a return conductor (e.g., a ground plane or other conductor), and an input impedance of the implantable antenna 705 can be controlled using the physical arrangement to provide a substantially conjugate match in a biological medium to an output impedance of an implantable telemetry circuit coupled to the implantable antenna 705, such as described in the commonly-assigned David Nghiem et al. U.S. Provisional Application 61/106,068, entitled "IMPEDANCE-CONTROLLED IMPLANTABLE TELEMETRY ANTENNA," filed on October 16, 2008.

In the example of FIG. 7, the implantable antenna 705 can be configured to be located in an implantable dielectric compartment having one or more ports or receptacles. In an example, the one or more ports or receptacles (e.g., typically one to five) can be configured to receive one or more leads or electrodes. Accordingly, the one or more leads or electrodes can be used to receive physiological information from a subject, or to deliver one or more therapies to the subject. In an example, the implantable antenna 705 can be configured to avoid the one or more ports or receptacles, or one or more other conductors in the implantable dielectric compartment.

In the example of FIG. 7, the implantable antenna 705 can reside in more than one plane. Further, one or more the major segments (e.g., the first major segment 706, the second major segment 707, or the third major segment 708) can be configured to be located along or near a side of the implantable dielectric compartment, or along or near the top of the implantable dielectric compartment. In the example of FIG. 7, the first major segment 706 is configured to be located proximate a side of the implantable dielectric compartment (not shown), and the third major segment 708 is configured to be located proximate a top of the implantable dielectric compartment.

In an example, to provide a first specified operating frequency in the MICS band frequency range and a second specified operating frequency in the ISM band frequency range in a biological medium, the implantable antenna 705 can have an initial segment length of 24.35 mm, a first major segment length of 29.5mm, second and third major segment lengths of 30mm, a first transition length of 3.3mm, and a second transition length of 3.75mm. In other examples, one or more other lengths can be used to provide the same or similar operating frequencies, or to provide one or more other specified operating frequencies.

FIG. 8 illustrates generally an example of a relationship 800 between a return loss (in decibels) and frequency (in GHz) of the antenna configuration illustrated in FIG. 7. In this example, plot 801 illustrates generally that the antenna configuration illustrated in FIG. 7 has a fundamental mode frequency at approximately 420 MHz and a first higher-order mode frequency at approximately 1 GHz.

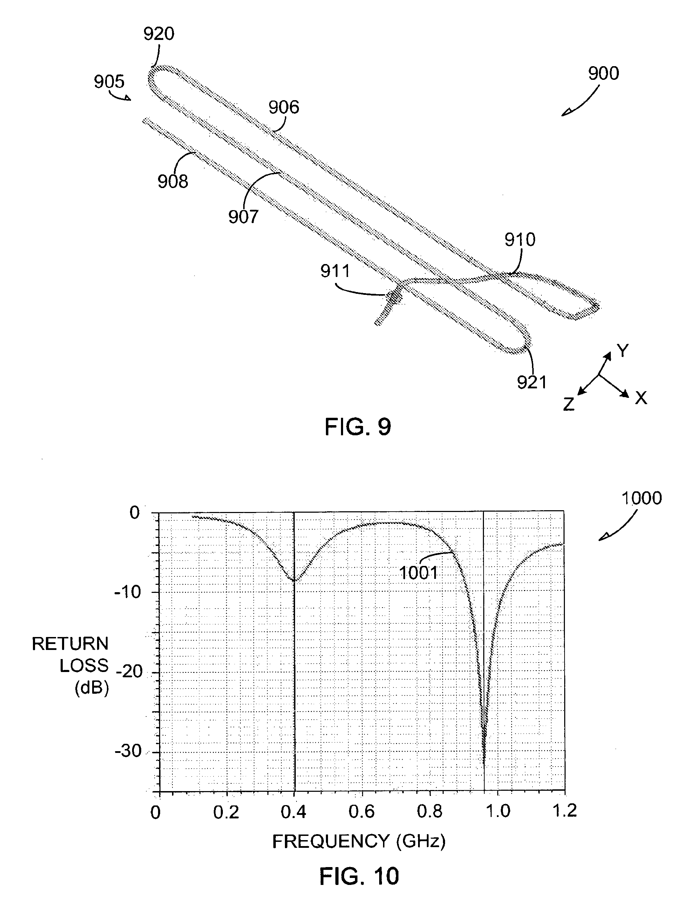

FIG. 9 illustrates generally an example of a system 900 including an implantable antenna 905 having a switchback portion including a first major segment 906, a second major segment 907, and a third major segment 908. In certain examples, the implantable antenna can include a first transition 920 configured to couple the first major segment 906 to the second major segment 907, and a second transition 921 configured to couple the second major segment 907 to the third major segment 908. In an example, the implantable antenna 905 can include an initial segment 910 different from the first major segment 906 of the switchback portion of the implantable antenna 905, the initial segment 910 configured to couple the first major segment 906 to an implantable telemetry circuit in an implantable device housing through feed-through 911.

In the example of FIG. 9, the first major segment 906, the second major segment 907, and the third major segment 908 can be configured to be located along one side of an implantable dielectric compartment, and the initial segment 910 can be configured to couple the first major segment 906 to the implantable telemetry circuit.

In certain examples, the first major segment 906, having a higher surface current at the fundamental mode than the other major segments, can be positioned farther from the implantable device housing or ground plane than one or more other major segment of the implantable antenna 905. In an example, positioning the first major segment 906 farther from the implantable device housing or ground plane can provide a lower amount of surface current cancellation between the first major segment 906 and the implantable device housing or ground plane, and accordingly, can least affect the radiation efficiency of the implantable antenna 905. In other examples, the first major segment 906 can be positioned closer to the implantable device housing or ground plane than one or more of the other major segments of the implantable antenna 905.

In an example, to provide a first specified operating frequency in the MICS band frequency range and a second specified operating frequency in the ISM band frequency range in a complex dielectric medium including a biological medium, the implantable antenna 905 can have an initial segment length of 24.8 mm, a first major segment length of 28 mm, a second major segment length of 26.5 mm, a third major segment lengths of 27 mm, and first and second transition lengths of 4.7 mm. In other examples, one or more other lengths can be used to provide the same or similar operating frequencies, or to provide one or more other specified operating frequencies.

In other examples, at least a portion of two or more major segments can be located equidistant to the medical device housing or ground plane.

FIG. 10 illustrates generally an example of a relationship 1000 between a return loss (in decibels) and frequency (in GHz) of the antenna configuration illustrated in FIG. 9. In this example, plot 1001 illustrates generally that the antenna configuration illustrated in FIG. 9 has a fundamental mode frequency at approximately 400 MHz and a first higher-order mode frequency at approximately 960 MHz.

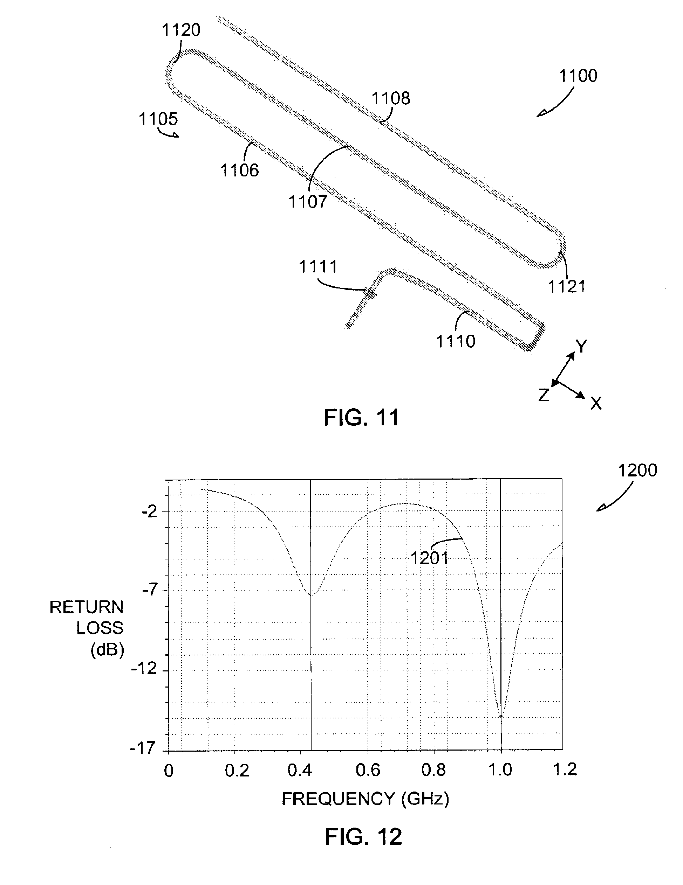

FIG. 11 illustrates generally an example of a system 1100 including an implantable antenna 1105 having a switchback portion including a first major segment 1106, a second major segment 1107, and a third major segment 1108. In certain examples, the implantable antenna can include a first transition 1120 configured to couple the first major segment 1106 to the second major segment 1107, and a second transition 1121 configured to couple the second major segment 1107 to the third major segment 1108. In an example, the implantable antenna 1105 can include an initial segment 1110 different from the first major segment 1106 of the switchback portion of the implantable antenna 1105, the initial segment 1110 configured to couple the first major segment 1106 to an implantable telemetry circuit in an implantable device housing through feed-through 1111.

In the example of FIG. 11, the first major segment 1106 can be configured to be located closer to an implantable device housing or ground plane than one or more other major segment of the implantable antenna 1105. In an example, to provide a first specified operating frequency in the MICS band frequency range and a second specified operating frequency in the ISM band frequency range in a complex dielectric medium including a biological medium, the implantable antenna 1105 can have an initial segment length of 22.5 mm, a first major segment length of 28 mm, a second major segment length of 26.5 mm, a third major segment lengths of 27 mm, and first and second transition lengths of 4.7 mm. In other examples, one or more other lengths can be used to provide the same or similar operating frequencies, or to provide one or more other specified operating frequencies.

FIG. 12 illustrates generally an example of a relationship 1200 between a return loss (in decibels) and frequency (in GHz) of the antenna configuration illustrated in FIG. 11. In this example, plot 1201 illustrates generally that the antenna configuration illustrated in FIG. 11 has a fundamental mode frequency at approximately 430 MHz and a first higher-order mode frequency at approximately 1000 MHz.

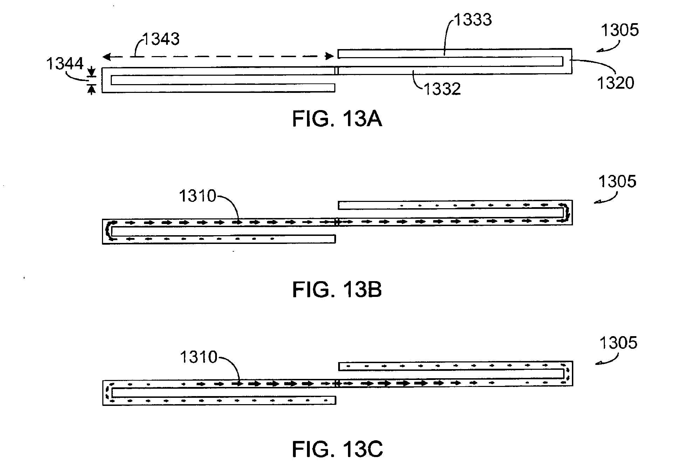

FIG. 13A illustrates generally an example of a two-segment folded dipole antenna 1305 (such as illustrated in the example of FIG. 4A). In an example, the two-segment dipole antenna 1305 can include two major segments defining a switchback portion. In the example of FIG. 13A, the switchback portion includes a first major segment 1332 and a second major segment 1333, the first major segment 1332 coupled to the second major segment 1333 using a first transition 1320. In an example, each major segment can have a fourth length 1343, and the first transition 1320 can have a fifth length 1344. In an example, the first length 440 of FIG. 4A can relate to the fourth length 1343 and the fifth length 1344 of FIG. 13A, where:

$$\textit{the first length 440} \cong \textit{(2 x the fourth length 1343)} + \textit{the fifth length 1344.}$$

In other examples, one or more of the major segments or the transition can include one or more other lengths, depending on the desired operating frequencies.

In an example, an unfolded dipole antenna as illustrated in FIG. 4A can have a fundamental mode frequency (e.g., approximately 315 MHz) providing a higher-order mode frequency (e.g., a second higher-order mode frequency) in the GPS band frequency range (e.g., 1572-1578 MHz). As illustrated in FIG. 13A, the first transition can be placed along the length of the two-segment folded dipole antenna 1305 to alter the fundamental mode of the two-segment folded dipole antenna 1305. In an example, the first transition 1320 can be placed to alter the surface current distribution in the switchback portion at the fundamental mode to provide a first desired operating frequency in the MICS band frequency range and to minimize changes to the surface current distribution in the switchback portion at the higher-order mode to provide a second desired operating frequency in the GPS band frequency range. In an example, the first length of the first major segment 1332 can correspond to one-eighth of an effective wavelength of the fundamental mode frequency of the unfolded antenna (e.g., illustrated in FIG. 4A) providing the higher-order mode frequency in the GPS band frequency range. In this example, the first length of the first major segment 1332 can correspond to one-eight of an effective wavelength of approximately 315 MHz.

In other examples, one or more of the fundamental or higher-order modes can be used or altered using the two-segment switchback portion to provide one or more other desired operating frequencies. In this example, the one or more other desired operating frequencies can be provided using one or more different major segment lengths, transition lengths, etc. In other examples, both of the fundamental mode and one or more higher-order modes can be altered to provide the desired operating frequencies.

FIG. 13B illustrates generally an example of a first surface current distribution 1310 along a two-segment folded dipole antenna 1305 at a fundamental mode of the two-segment folded dipole antenna 1305.

FIG. 13C illustrates generally an example of a second surface current distribution 1311 along a two-segment folded dipole antenna 1305 at a second higher-order mode of the two-segment folded dipole antenna 1305.

As illustrated in the example of FIG. 13B, at a fundamental mode, the direction of the first surface current distribution 1310 in a first major segment of the two-segment folded dipole antenna 1305 generally opposes the direction of the first surface current distribution 1310 in a second major segment of the two-segment folded dipole antenna 1305. Accordingly, surface current cancellation can occur, affecting the fundamental mode frequency of the two-segment folded dipole antenna 1315.

However, as illustrated in the example of FIG. 13C, at the second higher-order mode, the direction of the second surface current distribution 1311 in a first major segment of the two-segment folded dipole antenna 1305 does not substantially oppose the direction of the second surface current distribution 1311 in a second major segment of the two-segment folded dipole antenna 1305. Here, less surface current cancellation occurs, minimizing the affect of the first transition on the first higher-order mode of the two-segment folded dipole antenna 1315.

Accordingly, the switchback configuration of FIG. 13A affects the fundamental mode frequency more than the second higher-order mode frequency. In other examples, one or more other higher-order mode frequencies can be affected and utilized.

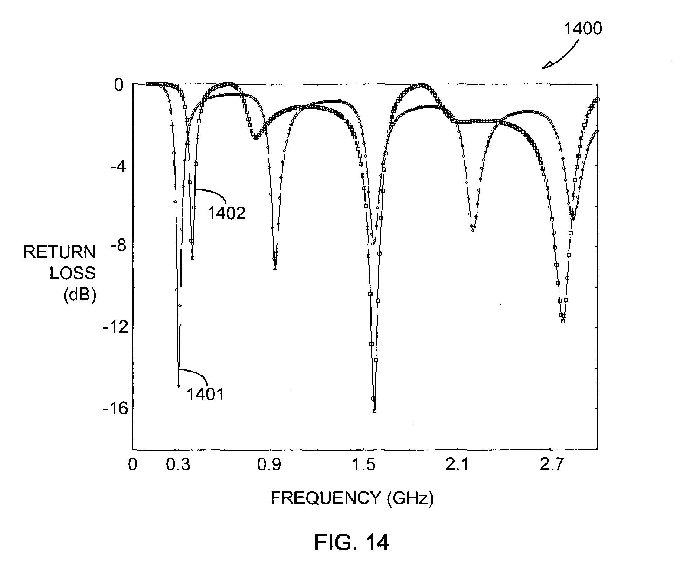

FIG. 14 illustrates generally an example of a relationship 1400 between a return loss (in decibels) and frequency (in GHz) of two example antenna configurations. In this example, plot 1401 illustrates generally an example of return loss characteristics of an unfolded dipole antenna (e.g., such as shown in FIG. 4A) having a fundamental mode frequency at approximately 300 MHz. Plot 1401 illustrates generally that higher-order modes, in this example, appear at odd multiples of the fundamental mode frequency (e.g., at approximately 900 MHz, at approximately 1.5 GHz, 2.1 GHz, 2.7 GHz, etc.). Further, in this example, plot 1402 illustrates generally an example of a folded dipole antenna having a switchback portion (e.g., such as shown in FIG. 14A) having a second higher-order mode frequency at approximately 1.6GHz, but having a fundamental mode frequency at approximately 400 MHz.

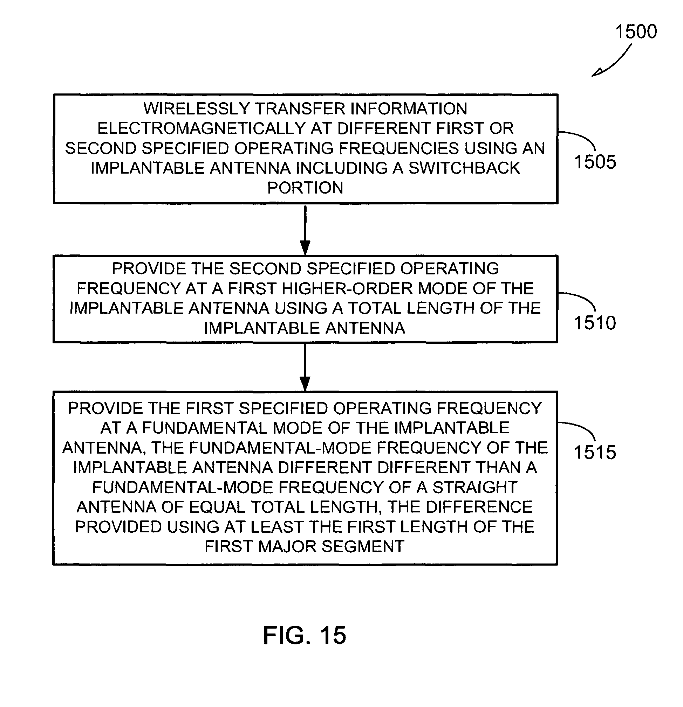

FIG. 15 illustrates generally an example of a method 1500 including wirelessly transferring information electromagnetically at different first or second specified operating frequencies. The wirelessly transferring information electromagnetically can include wirelessly sending or receiving information electromagnetically (e.g., sending and receiving concurrently, using one or more specified operating frequencies). In an example, an implantable antenna can be driven in a transmit mode of an implantable telemetry circuit, or information from an implantable antenna can be received in a receive mode of the implantable telemetry circuit. In an example, the driving or the receiving can include using different first or second specified operating frequencies. In certain examples, the first specified operating frequency can be different than the second specified operating frequency.

At 1505, information can be wirelessly transferred electromagnetically at different first or second specified operating frequencies using an implantable antenna including a switchback portion. In an example, information can be wirelessly transferred at both the first and second specified operating frequencies. In other examples, information can be wirelessly transferred using one of the first or second specified operating frequency, but the implantable antenna can be configured to wirelessly transfer information at the other specified operating frequency.

At 1510, the second specified operating frequency (e.g., higher than the first specified operating frequency) can be provided at a first higher-order mode of the implantable antenna using a total length of the implantable antenna.

In an example, starting from an unfolded implantable antenna, the total length can be selected, defining a fundamental mode frequency that provides the second specified operating frequency using a first higher-order mode. The fundamental mode frequency of the unfolded antenna can be altered or changed using one or more transitions along the length of the unfolded implantable antenna defining a switchback portion in the implantable antenna.

In an example, the one or more transitions can alter or change the surface current distribution along the implantable antenna at the fundamental mode, and differently at one or more higher-order modes. In these examples, the surface current in the overlapping portions of the first and second major segments (or one or more other segments) of the higher-order mode can be oriented in the same direction, providing a relatively low amount of surface current cancellation between the multiple segments. Accordingly, in certain examples, the second specified operating frequency of the implantable antenna including the switchback portion can remain substantially similar to a higher-order mode frequency of an unfolded implantable antenna of equal total length.

In other examples, the direction of the surface current of the higher-order mode in the overlapping portions of the first and second major segments (or one or more other segments) of the implantable antenna can partially oppose each other, or can partially be oriented in the same direction. In an example, the more the surface current of the higher-order mode in the overlapping portions of the first and second major segments oppose each other, the more the second specified operating frequency can change. In certain examples, the total length of the implantable antenna, of the switchback portion of the implantable antenna, or of one or more portions of the implantable antenna (e.g., the initial segment, one or more major segment, one or more transition, etc.) can be altered (increased or decreased) to compensate for the opposing surface current in the first and second major segments (or one or more other segments) of the implantable antenna.

In an example, to provide a first specified operating frequency at approximately 403.5 MHz and a second specified operating frequency at approximately 915 MHz, the first length of the first major segment can include approximately one-eighth of an effective wavelength of the lower specified operating frequency.

At 1515, the first specified operating frequency can be provided (as described above) at a fundamental mode of the implantable antenna, wherein the fundamental mode frequency of the implantable antenna is different than a fundamental mode frequency of an unfolded antenna of equal total length, the difference provided using at least the first length of the first major segment.

In other examples, one or more other frequencies can be selected, and one or more other antenna lengths can be selected in order to provide wireless communication at two or more frequencies. Further, in certain examples, to tune the antenna, the present inventors have recognized that the length of the antenna can be lengthened to improve the transmission characteristics at one or more specified operating frequencies. For example, the total length of the implantable antenna can exceed one quarter of an effective wavelength of a first or lower desired operating frequency.

In an example, a desired operating frequency can be selected between two operating bands (e.g., to provide communication at both frequency bands surrounding the desired operating frequency). In an example, one of a first or second desired operating frequency can be selected between the ISM and SRD band ranges to provide communication at each band. In certain examples, a second desired operating frequency can be selected at one or more other operating bands (e.g., the MICS band range), or between two operating bands.

In certain examples, the implantable antenna can be configured to fit within an implantable dielectric compartment. In various examples, different dielectric compartments can have different shapes, sizes, or components within them. Accordingly, in certain examples, one or more of the portions of the implantable antenna (e.g., one or more of the initial segment, the major segments, the transitions, etc.) can be altered (e.g., lengthened or shortened) to provide communication at the desired operating frequencies. In an example, the initial segment of the implantable antenna can lengthened, and the third major segment of the switchback portion can be shortened accordingly. In other examples, one or more other portions can be altered. In certain examples, the total length of the implantable antenna can remain constant. However, in other examples, alterations to certain segments (e.g., the first major segment) can affect the operation (e.g., the resulting operating frequencies) more than others, and can be accounted for accordingly.

Additional Notes

The above detailed description includes references to the accompanying drawings, which form a part of the detailed description. Some of the drawings show, by way of illustration, specific embodiments in which the invention can be practiced. Such examples can include elements in addition to those shown and described. However, the present inventors also contemplate examples in which only those elements shown and described are provided.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In the appended claims, the terms "including" and "in which" are used as the plain-English equivalents of the respective terms "comprising" and "wherein." Also, in the following claims, the terms "including" and "comprising" are open-ended, that is, a system, device, article, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description, with each claim standing on its own as a separate embodiment. The scope of the invention should be determined with reference to the appended claims, along with the full scope of equivalents to which such claims are entitled.

**Claims**

1. A multi-band implantable telemetry system, comprising:

   an implantable antenna (305);
   an implantable telemetry circuit (206) coupled to the implantable antenna (305), the implantable telemetry circuit (206) configured to drive the implantable antenna (305) in a transmit mode and receive information from the implantable antenna (305) in a receive mode using at least one of a first specified operating frequency or a second specified operating frequency, the second specified operating frequency different than the first specified operating frequency;
   wherein the implantable antenna (305) is configured to wirelessly send information electromagnetically from within a biological medium or
   wirelessly receive information electromagnetically in the biological medium using the first and second specified operating frequencies, the implantable antenna (305) including a switchback portion including:

   a first major segment (332) having a first length;
   a second major segment (333) having a second length;
   a third major segment (334) having a third length; and
   wherein the first (332), second (333), and third (334) major segments are arranged in a switchback configuration;

   wherein the switchback portion of the implantable antenna (305) includes:

   a first transition (320) between the first (332) and second (333) major segments, the first transition (320) configured to couple a distal end of the first major segment (332) to a proximal end of the second major segment (333), the first transition (320) including a first turn, such that the geometrical direction from a proximal end of the first major segment (332) to the distal end of the first major segment (332) substantially opposes the geometrical direction from the proximal end of the second major segment (333) to a distal end of the second major segment (333); and
   a second transition (321) between the second (333) and third (334) major segments, the second transition (321) configured to couple a distal end of the second major segment (333) to a proximal end of the third major segment (334), the second transition (321) including a second turn, such that the geometrical direction from a proximal end of the second major segment (333) to the distal end of the second major segment (333) substantially opposes the geometrical direction from the proximal end of the third major segment 334) to a distal end of the third major segment (334);
   wherein a sum of the first length, the second length, and the third length comprises a total length;
   wherein the first specified operating frequency is provided at a fundamental mode of the implantable antenna (305), wherein the fundamental mode frequency of the implantable antenna (305) is different than the fundamental mode frequency of an unfolded antenna equal to the total length, the difference in fundamental mode frequency provided by at least the first length of the first major segment (332);
   wherein the second specified operating frequency is provided at a first higher-order mode of the implantable antenna (305) by a total length of the implantable antenna (305); and
   wherein the first length of the first major segment (332) corresponds to one-eighth of the effective wavelength of the first specified operating frequency.

2. The system of claim 1, wherein the first higher-order mode frequency of the implantable antenna (305) corresponds to the first higher-order mode frequency of the unfolded antenna of equal total length.

3. The system of any one of claims 1 through 2, wherein the first length of the first major segment (332) is configured to:

(1) optimize surface current cancellation between the first major segment (332) and the second major segment (333) at the fundamental mode of the implantable antenna (305) to provide the first specified operating frequency; and

(2) minimize surface current cancellation between the first major segment (332) and the second major segment (333) at the first higher-order mode of the implantable antenna (305) to provide the second specified operating frequency.

4. The system of claim 1, wherein each of the first length of the first major segment (332), the second length of the second major segment (333), and the third length of the third major segment (334) separately correspond to one-eighth of the effective wavelength of the first specified operating frequency.

5. The system of any one of claims 1 through 4, wherein the first major segment (332) is located farther from a ground plane (315) than the second major segment (333).

6. The system of any one of claims 1 through 4, wherein the first major segment (332) is located closer to a ground plane (315) than the second major segment (333).

7. The system of any one of claims 1 through 4, wherein at least a portion of the first major segment (332) is located equidistant to a ground plane (315) with at least a portion of the second major segment (333).

8. The system of any one of claims 1 through 7, wherein the implantable antenna (305) comprises a wire, and wherein at least a portion of the second major segment (333) is separated from at least a portion of the first major segment (332) by a first distance, wherein the first distance is larger, by at least a factor of 3, than a diameter of the wire comprising the antenna, and wherein the first length of the first major segment (332) is larger, by at least a factor of 5, than the first distance between the first (332) and second (333) major segments.

9. The system of any one of claims 1 through 8, wherein the first and second specified operating frequencies are selected from a list including at least one of:

(1) a Medical Implant Communications Service (MICS) band range extending from approximately 402 MHz to approximately 405 MHz;
(2) a Short Range Device (SRD) band range extending from approximately 862 MHz to approximately 870 MHz;
(3) a first Industrial-Scientific-Medical (ISM) band range extending from approximately 902 MHz to approximately 928 MHz;
(4) a second ISM band range extending from approximately 2400 MHz to approximately 2500 MHz; or
(5) a Personal Communication Service (PCS) band range extending from approximately 1850-1990 MHz.

10. The system of any one of claims 1 through 9, wherein the distal end of the first major segment (332) is coupled to the proximal end of the second major segment (333), and wherein the distal end of the second major segment (333) is coupled to the proximal end of the third major segment (334); and
wherein the switchback portion of the implantable antenna (305) is composed of a single continuous conductor extending from the proximal end of the first major segment (332) to the distal end of the third major segment (334).

11. The system of claim 10, wherein the implantable antenna (305) includes an initial segment (310) configured to couple the telemetry circuit (206) to the proximal end of the first major segment (332), and wherein the initial segment (310) is configured to control an input impedance of the implantable antenna (305) at least in part using a physical arrangement of at least a portion of the initial segment (310) with respect to a return conductor to provide a substantially conjugate match in the biological medium to an output impedance of the implantable telemetry circuit (206).

12. The system of any one of claims 1 through 11, including:

an implantable device housing (205) including the implantable telemetry circuit (206) and a conductive portion coupled to the implantable telemetry circuit (206);
an implantable dielectric compartment (207) coupled to the implantable device housing (205), the implantable dielectric compartment (207) including the first (332), second (333), and third (334) major segments of the switchback portion of the implantable antenna (305), the implantable dielectric compartment (207) having a height with respect to the surface of the implantable device housing (205), and having a length and width along the surface of the implantable device housing (205);

wherein the implantable antenna (305) is configured to wirelessly send information electromagnetically from within the implantable dielectric compartment (207) in the biological medium or wirelessly receive information electromagnetically in the implantable dielectric compartment (207) in the biological medium using the first and second specified operating frequencies;

wherein the first, second, and third lengths of the first (332), second (333), and third (334) major segments are each shorter than the length of the implantable dielectric compartment (207); and

wherein at least a portion of the second major segment (333) is separated from at least a portion of the first major segment (332) by a first distance, the first distance shorter than at least one of the height or width of the implantable dielectric compartment (207).

13. The system of claim 12, wherein at least one of the first (332), second (333), or third (334) major segments of the switchback portion of the implantable antenna (305) are located proximate at least one of a side or a top of the implantable dielectric compartment (207).

14. A method comprising:

driving an implantable antenna (305) in a transmit mode and receiving information from the implantable antenna (305) in a receive mode using at least one of a first specified operating frequency or a second specified operating frequency different than the first specified operating frequency;

wirelessly sending information electromagnetically at one of the first or second specified operating frequencies using the implantable antenna (305) from within a biological medium or wirelessly receiving information electromagnetically at one of the first or the second specified operating frequencies using the implantable antenna (305) in the biological medium, the implantable antenna (305) including a switchback portion, the switchback portion including a first major segment (332) having a first length, a second major segment (333) having a second length, and a third major segment (334) having a third length, the first (332), second (333), and third (33) major segments arranged in a switchback configuration,

wherein the switchback portion of the implantable antenna (305) includes:

a first transition (320) between the first (332) and second (333) major segments, the first transition (320) configured to couple a distal end of the first major segment (322) to a proximal end of the second major segment (333), the first transition (320) including a first turn, such that the geometrical direction from a proximal end of the first major segment (332) to the distal end of the first major segment (332) substantially opposes the geometrical direction from the proximal end of the second major segment (333) to a distal end of the second major segment (233); and

a second transition (321) between the second (333) and third (334) major segments, the second transition (321) configured to couple a distal end of the second major segment (333) to a proximal end of the third major segment (334), the second transition (321) including a second turn, such that the geometrical direction from a proximal end of the second major segment (333) to the distal end of the second major segment (333) substantially opposes the geometrical direction from the proximal end of the third major segment (334) to a distal end of the third major segment (334);

wherein a sum of the first length, the second length, and the third length comprises a total length;

providing the first specified operating frequency at a fundamental mode of the implantable antenna (305), wherein the fundamental mode frequency of the implantable antenna (305) is different than the fundamental mode frequency of an unfolded antenna of equal to the total length, the difference in fundamental mode frequency provided by at least the first length of the first major segment (332); and providing the second specified operating frequency at a first higher-order mode of the implantable antenna (305) by a total length of the implantable antenna (305);

wherein the first length of the first major segment (332) corresponds to one-eighth of the effective wavelength of the first specified operating frequency.

**Patentansprüche**

1. Ein implantierbares telemetrisches Mehrbandsystem umfassend:

eine implantierbare Antenne (305);
eine implantierbare Telemetrie Schaltung (206) die mit der implantierbaren Antenne (305) verbunden ist, wobei die implantierbare Telemetrie Schaltung (206) ist so ausgelegt ist, um die implantierbare Antenne (305) in einem Übertragungsmodus zu steuern und in einem Empfangsmodus Informationen von der implantierbaren Antenne (305) zu erhalten, unter Benutzung von mindestens einer ersten bestimmten Betriebs-Frequenz oder eine von der ersten verschiedene zweiten bestimmte Betriebs-Frequenz;

wobei die implantierbare Antenne (305) so ausgelegt ist um drahtlos Informationen elektromagnetisch aus einem biologischen Medium zu senden, oder drahtlos Informationen elektromagnetisch in das biologische Medium zu erhalten, unter Benutzung von der erste und zweite bestimmten Betriebsfrequenz, wobei die implantierbare Antenne (305) mit einem serpentinartigem Bereich versehen ist, umfassend:

ein erstes Hauptsegment (332) mit einer ersten Länge;

ein zweites Hauptsegment (333) mit einer zweiten Länge;

ein drittes Hauptsegment (334) mit einer dritten Länge; wobei die ersten (332), die zweiten (333) und die dritten (334) Hauptsegmente in einer serpentinartigen Konfiguration angeordnet sind; und

wobei der serpentinartige Bereich der implantierbaren Antenne (305) umfasst:

eine erste Überleitung (320) zwischen dem ersten (332) und dem zweiten (333) Hauptsegment, wobei die erste Überleitung (320) so ausgelegt ist, um ein entferntes Ende des ersten Hauptsegments (332) mit einem nahen Ende des zweiten Hauptsegments (333) zu verbinden, wobei die erste Überleitung (320) eine erste Windung umfasst, so dass die geometrische Richtung von einem nahen Ende des ersten Hauptsegments (332) in Richtung zu dem entfernten Ende des ersten Hauptsegments (332) im Wesentlichen entgegengesetzt ist zu der geometrischen Richtung, die von dem nahen Ende des zweiten Hauptsegments (333) in Richtung zu einem entfernten Ende des zweiten Hauptsegments (333) verläuft; und

eine zweite Überleitung (321) zwischen dem zweiten (333) und dem dritten (334) Hauptsegment, wobei die zweite Überleitung (321) so ausgelegt ist, um ein entferntes Ende des zweiten Hauptsegments (333) mit einem nahen Ende des dritten Hauptsegments (334) zu verbinden, wobei die zweite Überleitung (321) eine zweite Windung umfasst, so dass die geometrische Richtung von einem nahen Ende des zweiten Hauptsegments (333) in Richtung zu dem entfernten Ende des ersten Hauptsegments (333) im Wesentlichen entgegengesetzt ist, zu der geometrischen Richtung, die von dem nahen Ende des dritten Hauptsegments (334) in Richtung zu einem entfernten Ende des dritten Hauptsegments (334) verläuft;

wobei eine Summe aus der ersten Länge, der zweiten Länge und der dritten Länge eine Gesamtlänge umfasst;

wobei die erste bestimmte Betriebsfrequenz bei einer Fundamentalmode der implantierbaren Antenne (305) bereitgestellt wird, wobei die Fundamentalmodenfrequenz der implantierbaren Antenne (305) verschieden ist von der Fundamentalmodenfrequenz einer entfalteten, der Gesamtlänge entsprechenden Antenne ist, wobei die Differenz in der Fundamentalmodenfrequenz von der mindestens ersten Länge des ersten Hauptsegments (332) bereitgestellt wird;

wobei die zweite bestimmte Betriebsfrequenz bei einer ersten Mode höherer Ordnung der implantierbaren Antenne (305) durch eine Gesamtlänge der implantierbaren Antenne (305) bereitgestellt wird; und

wobei die erste Länge des ersten Hauptsegments (332) einem Achtel der effektiven Wellenlänge der ersten bestimmten Betriebsfrequenz entspricht.

2. Das System nach Anspruch 1, wobei die erste Mode höherer Ordnung der implantierbaren Antenne (305) der Frequenz der ersten Mode höherer Ordnung entspricht, die von der entfalteten, mit einer gleichen Gesamtlänge der Antenne bereitgestellt wird.

3. Das System nach irgendeinem der Ansprüche 1 oder 2, wobei die erste Länge des ersten Hauptsegments (332) so konfiguriert ist um:

(1) die Oberflächenstrom-Aufhebung zwischen dem ersten Hauptsegment (332) und dem zweiten Hauptsegment (333) zu optimieren, in der Fundamentalmode der implantierbaren Antenne (305), um die erste bestimmte Betriebsfrequenz bereitzustellen; und

(2) die Oberflächenstrom-Aufhebung zwischen dem ersten Hauptsegment (332) und dem zweiten Hauptsegment (333) zu minimieren in der Fundamentalmode der implantierbaren Antenne (305), um die zweite bestimmte Betriebsfrequenz bereitzustellen.

4. Das System nach Anspruch 1, wobei jede der ersten Länge des ersten Hauptsegments (332), der zweiten Länge des zweiten Hauptsegments (333) und der dritten Länge des dritten Hauptsegments (334) jeweils einem Achtel der effektiven Wellenlänge der ersten bestimmten Betriebsfrequenz entsprechen.

**5.** Das System nach irgendeinem der Ansprüche 1 bis 4, wobei das erste Hauptsegment (332) weiter von einer Groundplane (315) entfernt ist als das zweite Hauptsegment (333).

**6.** Das System nach irgendeinem der Ansprüche 1 bis 4, wobei das erste Hauptsegment (332) näher an einer Groundplane (315) ist als das zweite Hauptsegment (333).

**7.** Das System nach irgendeinem der Ansprüche 1 bis 4, wobei der letzte Teil des ersten Hauptsegments (332) gleich weit entfernt ist von einer Groundplane (315) wie mindestens ein Teil des zweiten Hauptsegments (333).

**8.** Das System nach irgendeinem der Ansprüche 1 bis 7, wobei die implantierbare Antenne (305) ein Draht umfasst, und wobei mindestens ein Teil des zweiten Hauptsegments (333) mit einem ersten Abstand von mindestens einem Teil des ersten Hauptsegments (332) getrennt ist, wobei der erste Abstand um mindestens eine Faktor Drei grösser ist als der Durchmesser des Drahtes, welcher die Antenne umfasst, und wobei die erste Länge des ersten Hauptsegments (332) grösser ist um mindestens einen Faktor 5 als der erste Abstand zwischen dem ersten (332) und dem zweiten (333) Hauptsegment.

**9.** Das System nach irgendeinem der Ansprüche 1 bis 8, wobei die erste und die zweite bestimmte Betriebsfrequenz aus einer Liste so ausgewählt sind, dass sie mindestens eines von folgendem enthalten:

> (1) ein Medical Implant Communication Service (MICS) Band, das sich von ungefähr 402 MHz bis ungefähr 405 MHz erstreckt;
> (2) ein Kurzstreckenfunk (SRD) deren Band sich von ungefähr 862 MHz bis ungefähr 870 MHz erstreckt;
> (3) eine erstes Industrial-Scientific-Medical (ISM) Band, das sich von ungefähr 902 MHz bis ungefähr 928 MHz erstreckt;
> (4) eine zweites (ISM) Band, das sich von ungefähr 2400 MHz bis ungefähr 2500 MHz erstreckt; oder
> (5) Personal Communication Service (PCS) Band, das sich von 1850-1990 MHz erstreckt;

**10.** Das System nach irgendeinem der Ansprüche 1 bis 9, wobei das entfernte Ende des ersten Hauptsegments (332) mit dem nahen Ende des zweiten Hauptsegments (333) verbunden ist, und wobei das entfernte Ende des zweiten Hauptsegments (333) mit dem nahen Ende des dritten Hauptsegments (334) verbunden ist, und
wobei der serpentinartige Bereich der implantierbaren Antenne (305) aus einem einzigen durchgehenden Leiter besteht, der sich von dem nahen Ende des ersten Hauptsegments (332) bis zu dem entfernten Ende des dritten Hauptsegments (334) erstreckt.

**11.** Das System nach Anspruch 10, wobei die implantierbare Antenne (305) ein Anfangssegment (310) beinhaltet, welches so ausgelegt ist um, die Telemetrie Schaltung (206) an das nahe Ende des ersten Hauptsegments (332) zu koppeln, und wobei das Anfangssegment (310) so ausgelegt ist um eine Eingangsimpedanz der implantierbaren Antenne (305) zu kontrollieren, unter Verwendung von zumindest zum Teil einer physikalischen Anordnung, wobei diese mindestens einem Teil des Anfangssegments (310) in Bezug auf einen Rückleiters umfasst, um ein im Wesentlichen konjugierte Anpassung zu einer Ausgangsimpedanz der implantierbaren Telemetrie Schaltung (206) in dem biologischen Medium bereitzustellen.

**12.** Das System nach irgendeinem der Ansprüche 1 bis 11, umfasst:

> ein Gehäuse für ein implantierbares Gerät (205) das die implantierbare Telemetrie Schaltung (206) und einen an die implantierbare Telemetrie Schaltung (206) gekoppelten leitenden Teil beinhaltet,
> ein implantierbares dielektrisches Fach (207) welches an das Gehäuse für das implantierbare Gerät gekoppelt ist, wobei das implantierbare dielektrische Fach (207) umfasst die ersten (332) zweiten (333) und dritten (334) Hauptsegmente des serpentinartigen Bereiches der implantierbaren Antenne (305), wobei das implantierbare dielektrische Fach (207) eine Höhe bezüglich der Oberfläche des Gehäuses für ein implantierbares Gerät (205) hat, sowie eine Länge und Breite entlang der Oberfläche des Gehäuses für ein implantierbares Gerät (205) hat;
> wobei die implantierbare Antenne (305) so ausgelegt ist, um Informationen drahtlos elektromagnetisch aus einem implantierbaren dielektrischen Fach (207) in einem biologische Medium zu senden, oder um drahtlos elektromagnetisch Informationen zu empfangen in das implantierbare dielektrische Fach in dem biologischen Medium unter Verwendung der ersten und zweiten bestimmten Betriebsfrequenzen;
> wobei die erste, zweite und dritte Länge des ersten (332), zweiten (333) und des dritten (334) Hauptsegments jeweils kürzer als die Länge des implantierbaren dielektrischen Fachs (207) ist; und wobei mindestens ein Teil des zweiten Hauptsegments (333) durch einem ersten Abstand von mindestens einem Teil des ersten Hauptsegments (332) getrennt ist, wobei der erste Abstand kürzer als mindestens eines von der Höhe oder Breite des implantierbaren dielektrischen Fachs (207) ist.

**13.** Das System nach Anspruch 12, wobei mindestens eines des ersten (332), des zweiten (333), oder des dritten (334) Hauptsegmente des serpentinartigen Bereiches der implantierbaren Antenne (305) mindestens einem von einer Seite oder Oberseite des implantierbaren dielektrischen Fachs (207) nahe angeordnet sind.

**14.** Ein Verfahren, welches umfasst:

betreiben einer implantierbaren Antenne (305) in einem Übertragungsmodus und Empfangen von Informationen von der Implantierbaren Antenne (305) in einem Empfangsmodus unter Benutzung von mindestens einer der ersten bestimmten Betriebsfrequenz oder der von der ersten verschiedenen zweiten bestimmten Betriebsfrequenz;

drahtloses elektromagnetisches versenden von Informationen in einer der ersten oder zweiten bestimmten Betriebsfrequenz unter Verwendung der implantierbaren Antenne (305) aus einem biologischen Medium heraus, oder drahtloses elektromagnetisches Empfangen von Informationen in einer der ersten oder zweiten bestimmten Betriebsfrequenz unter Verwendung der implantierbaren Antenne (305) in einem biologischen Medium, wobei die implantierbare Antenne (305) einen serpentinartigen Bereich umfasst, wobei der serpentinartige Bereich ein erstes Hauptsegment (332) mit einer ersten Länge, ein zweites Hauptsegment (333) mit einer zweiten Länge, ein drittes Hauptsegment (334) mit einer dritten Länge umfasst, wobei das erste (332), das zweite (333) und das dritte (334) Hauptsegment in einer serpentinartigen Konfiguration angeordnet sind,

wobei der serpentinartige Bereich der implantierbaren Antenne (305) umfasst:

eine erste Überleitung (320) zwischen dem ersten (332) und dem zweiten (333) Hauptsegment, wobei die erste Überleitung (320) so ausgelegt ist um ein entferntes Ende des ersten Hauptsegments (332) mit einem nahem Ende des zweiten Hauptsegments (333) zu verbinden, wobei die erste Überleitung (320) eine erste Windung umfasst, so dass die geometrische Richtung von einem nahen Ende des ersten Hauptsegments (332) in Richtung zu dem entfernten Ende des ersten Hauptsegments (332) im Wesentlichen entgegengesetzt ist zu der geometrischen Richtung, die von dem nahen Ende des zweiten Hauptsegments (333) in Richtung zu einem entfernten Ende des zweiten Hauptsegments (333) ist verläuft; und

eine zweite Überleitung (321) zwischen dem zweitem (333) und dem dritten (334) Hauptsegment, wobei die zweite Überleitung (321) ist so ausgelegt ist um ein entferntes Ende des zweiten Hauptsegments (333) mit einem nahem Ende des dritten Hauptsegments (334) zu verbinden, wobei die zweite Überleitung (321) eine zweite Windung umfasst, so dass die geometrische Richtung von einem nahen Ende des zweiten Hauptsegments (333) in Richtung zu dem entfernten Ende des ersten Hauptsegments (333) im Wesentlichen entgegengesetzt ist zu der geometrischen Richtung, die von dem nahen Ende des dritten Hauptsegments (334) in Richtung zu einem entfernten Ende des dritten Hauptsegments (334) verläuft;

wobei eine Summe aus der ersten Länge, der zweiten Länge und der dritten Länge eine Gesamtlänge umfasst;

bereitstellen der ersten bestimmte Betriebsfrequenz bei einer Fundamentalmode der implantierbaren Antenne (305), wobei die Fundamentalmodenfrequenz der implantierbaren Antenne (305) verschieden ist von der Fundamentalmodenfrequenz einer entfalteten, der Gesamtlänge entsprechenden Antenne ist, wobei die Differenz in der Fundamentalmodenfrequenz von der mindestens ersten Länge des ersten Hauptsegments (332) bereitgestellt wird; und

wobei die zweite bestimmte Betriebsfrequenz bei einer ersten Mode höherer Ordnung der implantierbaren Antenne (305) durch eine Gesamtlänge der implantierbaren Antenne (305) bereitgestellt wird;

wobei die erste Länge des ersten Hauptsegments (332) einem Achtel der effektiven Wellenlänge der ersten bestimmten Betriebsfrequenz entspricht.

## Revendications

**1.** Système de télémesure implantable multi-bande, comprenant :

une antenne implantable (305) ;

un circuit de télémesure implantable (206) couplé à l'antenne implantable (305), le circuit de télémesure implantable (206) étant configuré pour commander l'antenne implantable (305) dans un mode de transmission et recevoir des informations provenant de l'antenne implantable (305) dans un mode de réception en utilisant au moins une fréquence parmi une première fréquence de fonctionnement spécifiée et une seconde fréquence de fonctionnement spécifiée, la seconde fréquence de fonctionnement spécifiée étant différente de la première fréquence de fonctionnement spécifiée ;

où l'antenne implantable (305) est configurée pour envoyer sans fil des informations par voie électromagnétique à partir d'un milieu biologique ou pour recevoir sans fil des informations par voie électromagnétique dans le milieu biologique au moyen des première et seconde fréquences de fonctionnement spécifiées, l'antenne implantable (305) comprenant une portion en lacet comprenant :

un premier segment principal (332) ayant une première longueur ;
un deuxième segment principal (333) ayant une deuxième longueur ;
un troisième segment principal (334) ayant une troisième longueur ; et
où les premier (332), deuxième (333), et troisième (334) segments principaux sont agencés selon une configuration en lacet ;

où la portion en lacet de l'antenne implantable (305) comprend :

une première transition (320) entre les premier (332) et deuxième (333) segments principaux, la première transition (320) étant configurée pour coupler une extrémité distale du premier segment principal (332) à une extrémité proximale du deuxième segment principal (333), la première transition (320) comprenant un premier coude, de sorte que la direction géométrique d'une extrémité proximale du premier segment principal (332) à l'extrémité distale du premier segment principal (332) soit sensiblement opposée à la direction géométrique de l'extrémité proximale du deuxième segment principal (333) à l'extrémité distale du deuxième segment principal (333) ;

une deuxième transition (321) entre les deuxième (333) et troisième (334) segments principaux, la deuxième transition (321) étant configurée pour coupler une extrémité distale du deuxième segment principal (333) à une extrémité proximale du troisième segment principal (334), la deuxième transition (321) comprenant un deuxième coude, de sorte que la direction géométrique d'une extrémité proximale du deuxième segment principal (333) à l'extrémité distale du deuxième segment principal (333) soit sensiblement opposée à la direction géométrique de l'extrémité proximale du troisième segment principal (334) à l'extrémité distale du troisième segment principal (334) ;

où une somme de la première longueur, de la deuxième longueur et de la troisième longueur comprend une longueur totale ;

où la première fréquence de fonctionnement spécifiée est pourvue à un mode fondamental de l'antenne implantable (305), où la fréquence du mode fondamental de l'antenne implantable (305) est différente de la fréquence du mode fondamental d'une antenne non pliée ayant une même longueur totale, la différence de fréquence de mode fondamental étant fournie par au moins la première longueur du premier segment principal (332) ; où la seconde fréquence de fonctionnement spécifiée est fournie à un premier mode d'ordre supérieur de l'antenne implantable (305) par une longueur totale de l'antenne implantable (305) ; et

où la première longueur du premier segment principal (332) correspond à un huitième de la longueur d'onde effective de la première fréquence de fonctionnement spécifiée.

2. Système selon la revendication 1, dans lequel la première fréquence de mode d'ordre supérieur de l'antenne implantable (305) correspond à la première fréquence de mode d'ordre supérieur de l'antenne non pliée ayant la même longueur totale.

3. Système selon l'une quelconque des revendications 1 à 2, dans lequel la première longueur du premier segment principal (332) est configurée pour :

(1) optimiser l'annulation du courant de surface entre le premier segment principal (332) et le deuxième segment principal (333) au mode fondamental de l'antenne implantable (305) pour fournir la première fréquence de fonctionnement spécifiée ; et
(2) minimiser l'annulation du courant de surface entre le premier segment principal (332) et le deuxième segment principal (333) au premier mode d'ordre supérieur de l'antenne implantable (305) pour fournir la deuxième fréquence de fonctionnement spécifiée.

4. Système selon la revendication 1, dans lequel chacune de la première longueur du premier segment principal (332), de la deuxième longueur du deuxième segment principal (333) et de la troisième longueur du troisième segment principal (334) correspond séparément à un huitième de la longueur d'onde effective de la première fréquence de fonctionnement spécifiée.

**5.** Système selon l'une quelconque des revendications 1 à 4, dans lequel le premier segment principal (332) se trouve plus loin d'un plan de masse (315) que le deuxième segment principal (333).

**6.** Système selon l'une quelconque des revendications 1 à 4, dans lequel le premier segment principal (332) se trouve plus près d'un plan de masse (315) que le deuxième segment principal (333).

**7.** Système selon l'une quelconque des revendications 1 à 4, dans lequel au moins une partie du premier segment principal (332) se trouve à égale distance d'un plan de masse (315) avec au moins une partie du deuxième segment principal (333).

**8.** Système selon l'une quelconque des revendications 1 à 7, dans lequel l'antenne implantable (305) comprend un fil métallique, et dans lequel au moins une partie du deuxième segment principal (333) est séparée d'au moins une partie du premier segment principal (332) par une première distance, dans lequel la première distance est plus grande, d'au moins un facteur 3, qu'un diamètre du fil métallique comprenant l'antenne, et dans lequel la première longueur du premier segment principal (332) est plus grande, d'au moins un facteur 5, que la première distance entre les premier (332) et deuxième (333) segments principaux.

**9.** Système selon l'une quelconque des revendications 1 à 8, dans lequel les première et seconde fréquences de fonctionnement spécifiées sont sélectionnées à partir d'une liste comprenant au moins une plage parmi :

(1) une plage de bande du service de communication d'implants médicaux (MICS) s'étendant d'environ 402 MHz à environ 405 MHz ;
(2) une plage de bande de dispositif à courte portée (SRD) s'étendant d'environ 862 MHz à environ 870 MHz ;
(3) une première plage de bande pour appareils industriels, scientifiques et médicaux (ISM) s'étendant d'environ 902 MHz à environ 928 MHz ;
(4) une seconde plage de bande ISM s'étendant d'environ 2400 MHz à environ 2500 MHz ; ou
(5) une plage de bande de service de communication personnelle (PCS) s'étendant d'environ 1850 à 1990 MHz.

**10.** Système selon l'une quelconque des revendications 1 à 9, dans lequel l'extrémité distale du premier segment principal (332) est couplée à l'extrémité proximale du deuxième segment principal (333), et dans lequel l'extrémité distale du deuxième segment principal (333) est couplée à l'extrémité proximale du troisième segment principal (334) ;
et
dans lequel la partie en lacet de l'antenne implantable (305) est composée d'un unique conducteur continu s'étendant de l'extrémité proximale du premier segment principal (332) à l'extrémité distale du troisième segment principal (334).

**11.** Système selon la revendication 10, dans lequel l'antenne implantable (305) comprend un segment initial (310) configuré pour coupler le circuit de télémesure (206) à l'extrémité proximale du premier segment principal (332), et dans lequel le segment initial (310) est configuré pour commander une impédance d'entrée de l'antenne implantable (305) au moins en partie au moyen d'un agencement physique d'au moins une partie du segment initial (310) par rapport à un conducteur de retour pour fournir une correspondance sensiblement conjuguée dans le milieu biologique à une impédance de sortie du circuit de télémesure implantable (206).

**12.** Système selon l'une quelconque des revendications 1 à 11, comprenant :

un boîtier de dispositif implantable (205) comprenant le circuit de télémesure implantable (206) et une partie conductrice couplée au circuit de télémesure implantable (206) ;
un compartiment diélectrique implantable (207) couplé au boîtier de dispositif implantable (205),
le compartiment diélectrique implantable (207) comprenant les premier (332), deuxième (333) et
troisième (334) segments principaux de la partie en lacet de l'antenne implantable (305), le compartiment diélectrique implantable (207) ayant une certaine hauteur par rapport à la surface du boîtier de dispositif implantable (205), et ayant une longueur et une largeur le long de la surface du boîtier de dispositif implantable (205) ;
où l'antenne implantable (305) est configurée pour envoyer sans fil des informations par voie électromagnétique depuis l'intérieur du compartiment diélectrique implantable (207) dans le milieu biologique ou recevoir sans fil des informations par voie électromagnétique dans le compartiment diélectrique implantable (207) dans le milieu biologique au moyen des première et

deuxième fréquences de fonctionnement spécifiées ;

où les première, deuxième et troisième longueurs des premier (332), deuxième (333) et troisième (334) segments principaux sont chacune plus courtes que la longueur du compartiment diélectrique implantable (207) ; et

où au moins une partie du deuxième segment principal (333) est séparée d'au moins une partie du premier segment principal (332) par une première distance, la première distance étant plus courte qu'au moins une distance parmi la hauteur ou la largeur du compartiment diélectrique implantable (207).

13. Système selon la revendication 12, dans lequel au moins un des premier (332), deuxième (333), ou troisième (334) segments principaux de la partie en lacet de l'antenne implantable (305) sont situés à proximité d'au moins un côté ou un sommet du compartiment diélectrique implantable (207).

14. Procédé comprenant les étapes suivantes :

commander une antenne implantable (305) dans un mode de transmission et recevoir des informations provenant de l'antenne implantable (305) dans un mode de réception en utilisant au moins une fréquence parmi une première fréquence de fonctionnement spécifiée et une seconde fréquence de fonctionnement spécifiée différente de la première fréquence de fonctionnement spécifiée ;

envoyer sans fil des informations par voie électromagnétique à l'une des première ou seconde fréquences de fonctionnement spécifiées au moyen de l'antenne implantable (305) depuis un milieu biologique ou recevoir sans fil des informations par voie électromagnétique à l'une des première ou

seconde fréquences de fonctionnement spécifiées au moyen de l'antenne implantable (305) dans le milieu biologique, l'antenne implantable (305) comprenant une portion en lacet, la portion en lacet comprenant un premier segment principal (332) ayant une première longueur, un deuxième segment principal (333) ayant une deuxième longueur, et un troisième segment principal (334) ayant une troisième longueur, les premier (332), deuxième (333), et troisième (334) segments principaux étant agencés selon une configuration en lacet, où la portion en lacet de l'antenne implantable (305) comprend :

une première transition (320) entre les premier (332) et deuxième (333) segments principaux, la première transition (320) étant configurée pour coupler une extrémité distale du premier segment principal (332) à une extrémité proximale du deuxième segment principal (333), la première transition (320) comprenant un premier coude, de sorte que la direction géométrique d'une extrémité proximale du premier segment principal (332) à l'extrémité distale du premier segment principal (332) soit sensiblement opposée à la direction géométrique de l'extrémité proximale du deuxième segment principal (333) à l'extrémité distale du deuxième segment principal (333) ; et

une deuxième transition (321) entre les deuxième (333) et troisième (334) segments principaux, la deuxième transition (321) étant configurée pour coupler une extrémité distale du deuxième segment principal (333) à une extrémité proximale du troisième segment principal (334), la deuxième transition (321) comprenant un deuxième coude, de sorte que la direction géométrique d'une extrémité proximale du deuxième segment principal (333) à l'extrémité distale du deuxième segment principal (333) soit sensiblement opposée à la direction géométrique de l'extrémité proximale du troisième segment principal (334) à l'extrémité distale du troisième segment principal (334) ;

où une somme de la première longueur, de la deuxième longueur et de la troisième longueur comprend une longueur totale ;

délivrer la première fréquence de fonctionnement spécifiée à un mode fondamental de l'antenne implantable (305), où la fréquence du mode fondamental de l'antenne implantable (305) est différente de la fréquence du mode fondamental d'une antenne non pliée ayant une même longueur totale, la différence de fréquence de mode fondamental étant fournie par au moins la première longueur du premier segment principal (332) ; et

délivrer la seconde fréquence de fonctionnement spécifiée à un premier mode d'ordre supérieur de l'antenne implantable (305) par une longueur totale de l'antenne implantable (305) ;

où la première longueur du premier segment principal (332) correspond à un huitième de la longueur d'onde effective de la première fréquence de fonctionnement spécifiée.

IMPLANTABLE
TELEMETRY
CIRCUIT

110

105

100

FIG. 1

217
215
EXTERNAL
MODULE
EXTERNAL
TELEMETRY
CIRCUIT
216

200

IMPLANTABLE
DIELECTRIC
COMPARTMENT
207
201
210
202
IMPLANTABLE
TELEMETRY
CIRCUIT
206
IMPLANTABLE DEVICE
HOUSING
205

FIG. 2

FIG. 3A

FIG. 3B

FIG. 3C

440

FIG. 4A

405

410

FIG. 4B

405

411

FIG. 4C

405

FIG. 5A

FIG. 5B

FIG. 5C

FIG. 6

FIG. 7

FIG. 8

FIG. 9

FIG. 10

FIG. 11

FIG. 12

FIG. 13A

FIG. 13B

FIG. 13C

FIG. 14

1500

┌─────────────────────────────────────────────────┐
│ WIRELESSLY TRANSFER INFORMATION                  │
│ ELECTROMAGNETICALLY AT DIFFERENT FIRST OR        │
│ SECOND SPECIFIED OPERATING FREQUENCIES USING AN  │  1505
│ IMPLANTABLE ANTENNA INCLUDING A SWITCHBACK       │
│ PORTION                                          │
└─────────────────────────────────────────────────┘
                         │
                         ▼
┌─────────────────────────────────────────────────┐
│ PROVIDE THE SECOND SPECIFIED OPERATING           │
│ FREQUENCY AT A FIRST HIGHER-ORDER MODE OF THE    │
│ IMPLANTABLE ANTENNA USING A TOTAL LENGTH OF THE  │  1510
│ IMPLANTABLE ANTENNA                              │
└─────────────────────────────────────────────────┘
                         │
                         ▼
┌─────────────────────────────────────────────────┐
│ PROVIDE THE FIRST SPECIFIED OPERATING FREQUENCY  │
│ AT A FUNDAMENTAL MODE OF THE IMPLANTABLE         │
│ ANTENNA, THE FUNDAMENTAL-MODE FREQUENCY OF THE   │  1515
│ IMPLANTABLE ANTENNA DIFFERENT DIFFERENT THAN A   │
│ FUNDAMENTAL-MODE FREQUENCY OF A STRAIGHT         │
│ ANTENNA OF EQUAL TOTAL LENGTH, THE DIFFERENCE    │
│ PROVIDED USING AT LEAST THE FIRST LENGTH OF THE  │
│ FIRST MAJOR SEGMENT                              │
└─────────────────────────────────────────────────┘

FIG. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009228075 A1
- US 20090182426 A1
- EP 0814536 A2
- US 61106068 A, David Nghiem